# EUROPÄISCHE PATENTSCHRIFT

(11) **EP 2 757 905 B1**
(45) Veröffentlichungstag und Bekanntmachung des Hinweises auf die Patenterteilung: **19.08.2015**
(21) Anmeldenummer: 11761557.5
(22) Anmeldetag: 20.09.2011
(51) Int. Cl.: A23L 1/226, C11B 9/00, C07D 317/22, C07D 317/72

(54) **ACETALE UND KETALE ALS RIECH- UND AROMASTOFFE**
ACETALS AND KETALS AS FRAGRANCES AND FLAVORS
ACÉTALS ET CÉTALS EN TANT QUE MATIÈRES ODORANTES ET AROMATIQUES

(43) Veröffentlichungstag der Anmeldung: 30.07.2014
(73) Patentinhaber: Symrise AG, 37603 Holzminden (DE)
(72) Erfinder: HÖLSCHER, Bernd, 37620 Halle (DE)
(74) Vertreter: Eisenführ Speiser
(86) Internationale Anmeldenummer: PCT/EP2011/066355
(87) Internationale Veröffentlichungsnummer: WO 2013/041130

(56) Entgegenhaltungen:
- EP-A1- 0 028 780
- WO-A1-2007/004740
- JP-A- 58 039 680
- US-A- 4 735 932
- US-A1- 2002 068 075
- DATABASE WPI Week 197738 Thomson Scientific, London, GB; AN 1977-67751Y XP002678148, & JP 52 095669 A (NAGOYA CITY) 11. August 1977 (1977-08-11)

## Beschreibung

Die vorliegende Erfindung betrifft primär die Verwendung von Acetalen bzw. Ketalen der nachfolgenden Formel (I) als Riech- und/oder Aromastoffe. Die Erfindung betrifft zudem Riech- und/oder Aromastoffkompositionen enthaltend ein oder mehrere dieser Acetale bzw. Ketale, parfümierte und aromatisierte Artikel umfassend ein oder mehrere dieser Acetale bzw. Ketale sowie entsprechende Verfahren zum Vermitteln, Modifizieren und/oder Verstärken bestimmter Geruchs- und/oder Geschmacksnoten.

Trotz einer Vielzahl bereits vorhandener Riech- und Aromastoffe besteht in der Parfüm- und Aromenindustrie auch weiterhin ein genereller Bedarf an neuen Riech- und Aromastoffen. So besteht beispielsweise ein Bedarf an Riech- oder Aromastoffen, die in der Lage sind (in Riech- oder Aromastoffkompositionen) neben einer primären Duft- oder Geschmacksnote weitere interessante Noten zu erzeugen und mit ihren neuartigen bzw. originellen olfaktorischen Eigenschaften die Möglichkeiten des Parfümeurs bzw. Flavoristen zu erweitern. Insbesondere besteht ein Interesse an Riechstoffen mit Duftnoten, welche in der Lage sind, eine harmonische Kombination mit blumigen und/oder fruchtigen Riechstoffen einzugehen. Vorzugsweise sollte dabei eine Überlagerung der unterschiedlichen geruchlichen Aspekte und Noten erfolgen, um dadurch einen insgesamt komplexen Geruchseindruck zu erzeugen.

Für die Kreation neuartiger Kompositionen besteht ständiger Bedarf an Riech- und Aromastoffen mit besonderen sensorischen Eigenschaften, die geeignet sind, als Grundlage für die Komposition von neuartigen Parfüms oder Aromen mit komplexem sensorischen Charakter zu dienen. Bevorzugte gesuchte Riech- und Aromastoffe sollten neben einer bestimmten Note weitere Noten und Aspekte aufweisen, die ihnen Charakter und Komplexität verleihen.

Mit "Galbanum" wird die in der Parfümerie wichtige grüne Geruchsrichtung des Galbanumöls bezeichnet. Galbanumöl ist ein etherisches Öl, welches typischerweise mittels Wasserdampfdestillation aus dem Gummiharz von *Ferula galbaniflua* oder *Ferula rubricaulis* gewonnen wird.

Das Geruchsbild von Galbanumöl ist komplex. Der charakteristische Geruch von Galbanumöl weist neben einer grünen Hauptnote (die als an grüne Äpfel, grüne Paprika, grüne Erbsen(schoten) erinnernd beschrieben wird) ferner frische, krautige, fruchtige (zumeist als Ananas beschrieben), holzige und würzige Noten sowie erdige, fettige, metallische und balsamische Aspekte auf.

Galbanumöl enthält neben eine Vielzahl verschiedener Monoterpene, Sesquiterpene, Terpenalkohole, Sesquiterpenalkohole, Terpenalkoholenacetate und Sesquiterpenalkoholacetate weitere Verbindungen. Diese weiteren Verbindungen, die lediglich in geringer Konzentration im Galbanumöl enthalten sind, weisen gänzlich anderen Strukturen auf als die soeben genannten, tragen jedoch auf Grund ihrer niedrigen Geruchsschwellenwerte in erheblichem Ausmaß zu dem Geruchsbild des Galbanumöls bei. Solche charakteristischen Minderbestandteile sind beispielsweise 1,3,5-Undecatrien (auch Galbanolen genannt) oder 2-Methoxy-3-isobutylpyrazin.

Bekannte Riechstoffe mit galbanumartigem Geruch sind Allylamylglycolat, welches einen stark-fruchtigen Galbanumgeruch mit Ananas-Aspekten aufweist, Al-lyl(cyclohexyloxy)acetat, ein hell nach Galbanum und Ananas riechender Stoff mit grünblättrigen, wässrigen, metallischen und beerigen Nuancen, und 1-(5,5-Dimethyl-1-cyclohexen-1-yl)-4-penten-1-on (manchmal auch Galbanum-Keton genannt, kommerziell beispielsweise unter den Produktnamen Dynascone oder Galbascone erhältlich), welches einen intensiven an Galbanum erinnernden grünen Geruch und fruchtige (Ananas), blumige (Hyazinthe), erdige, metallische sowie ozonige Noten aufweist.

US-A-2002/068075 offenbart einen parfümierten Artikel umfassend eine Citral-Acetal-Verbindung.

EP-A-0028780 offenbart eine Riechstoffkomposition umfassend 2-Butyl-1,4-dioxaspiro-(4,4)-nonan.

Es besteht in der Duft- und Aromenindustrie weiterhin der Bedarf an galbanumartigen Stoffen.

Die Suche nach geeigneten galbanumartigen Stoffen, die zur vorliegenden Erfindung führte, wurde durch folgende Sachverhalte erschwert:
- die Mechanismen der Geruchswahrnehmung sind nicht ausreichend bekannt;
- die Zusammenhänge zwischen der speziellen Geruchswahrnehmung einerseits und der chemischen Struktur des zugehörigen Riechstoffs andererseits sind nicht hinreichend erforscht;
- häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten Riech- und Aromastoffen hängt deshalb stark von der Intuition des Suchenden ab.

Die primäre Aufgabe bestand nun darin, Riech- und/oder Aromastoffe zu finden, die ein interessantes, vorzugsweise komplexes, und originelles sensorisches Profil aufweisen und sich als Riechstoffe für den Einsatz in der Parfümerie bzw. als Aromastoffe zur Aromatisierung von verzehrbaren Zubereitungen eignen.

Es wurden im Rahmen der vorliegenden Erfindung insbesondere Stoffe gesucht, die eine, mehrere oder sämtliche der der Noten grün, krautig, frisch, fruchtig, blumig, holzig, süß, erdig, fettig, metallisch und balsamisch aufweisen bzw. vermitteln, modifizieren und/oder verstärken können. Insbesondere sollten Stoffe mit Noten gefunden werden, welche vorzugsweise zumindest einer der Noten grün und/oder fruchtig (dabei vorzugsweise in Richtung Ananas) aufweisen und bevorzugt galbanumartig riechen bzw. eine Galbanum-Note vermitteln können.

Die gesuchten Stoffe sollten die Herstellung von neuartigen Riech- oder Aromastoffkompositionen mit besonderen geruchlichen bzw. geschmacklichen Noten und Aspekten ermöglichen. Von Vorteil wären dabei solche Stoffe, welche insbesondere zur Kombination mit weiteren Riech- oder Aromastoffen geeignet sind, die eine holzige und/oder blumige und/oder fruchtige Note aufweisen.

Daneben sollten die diese primäre Aufgabe erfüllenden Riech- und/oder Aromastöffe vorzugsweise über ihre primären, nämlich geruchlichen, Eigenschaften hinaus zusätzliche positive Sekundäreigenschaften besitzen, wie z.B. eine hohe Stabilität unter bestimmten Anwendungsbedingungen, eine hohe Ausgiebigkeit, ein gutes Haftungsvermögen, eine hohe Substantivität, geruchs- und/oder geschmacksverstärkende Eigenschaften aufweisen (sogenannte Booster- oder Enhancer-Wirkung) und/oder in Kombination mit anderen Riech- und/oder Aromastoffen deren Natürlichkeit, Frische, Fülle, (Strahl)Kraft und/oder Ausstrahlung abzurunden, so dass sensorisch bemerkenswerte Effekte erzielt werden können.

Erfindungsgemäß gelöst wird die primäre gestellte Aufgabe durch die Verwendung von Verbindungen der Formel (I) 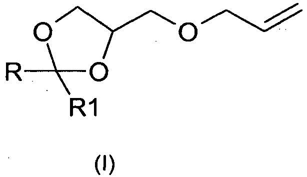 wobei R und R1
- jeweils unabhängig voneinander Wasserstoff oder einen organischen Rest mit 1 bis 15 Kohlenstoffatomen bedeuten
oder
- zusammen mit dem Kohlenstoffatom, an das sie gebunden sind, einen Ring bilden,
als Riech- oder Aromastoff.

Die erfindungsgemäßen bzw. erfindungsgemäß einzusetzenden Verbindungen der Formel (I) können in beliebiger optisch aktiven Form wie auch als beliebige Mischung von Stereoisomeren vorliegen, beispielsweise als Diastereomerengemisch oder als Racemat.

Die Verbindungen der Formel (I) verfügen über ganz eigenständige olfaktorische Eigenschaften, die sich deutlich von den bekannten Riechstoffen abheben und diese auch übertreffen. Die Eignung der Verbindungen der Formel (I) als Riech- oder Aromastoffe war bislang nicht bekannt. Es ist daher besonders überraschend, dass auf dem bereits gut untersuchten Gebiet der Acetale und Ketale wertvolle Riechstoffe mit interessanten und komplexen Geruchseigenschaften gefunden werden konnten.

Vorzugsweise betrifft die vorliegende Erfindung die Verwendung einer Verbindung der Formel (I) als Riech- oder Aromastoff
(i) mit Galbanum-Note
   und/oder
(ii) zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, süß, erdig, fettig, metallisch und balsamisch, wobei vorzugsweise zumindest einer der Noten grün und/oder fruchtig vermittelt, modifiziert und/oder verstärkt wird.

Die Verbindungen der Formel (I) eignen sich insbesondere
(ii) zum Vermitteln einer, zweier oder mehrerer Geruchs- und/oder Geschmacksnoten ausgewählt aus der Gruppe bestehend aus den Noten grün, krautig, frisch, fruchtig, blumig, holzig, süß, erdig, fettig, metallisch und balsamisch.

Bevorzugte Verbindungen der Formel (I) eignen sich
(ii) zum Vermitteln einer grünen und/oder fruchtigen (dabei vorzugsweise ananasartigen) Geruchs- und/oder Geschmacksnote und zusätzlich einer, zweier oder mehrerer Geruchs- und/oder Geschmacksnoten ausgewählt aus der Gruppe bestehend aus den Noten krautig, frisch, blumig, holzig, süß, erdig, fettig, metallisch und balsamisch.

Weiter bevorzugte Verbindungen der Formel (I) eignen sich
(ii) zum Vermitteln einer grünen und fruchtigen (dabei vorzugsweise ananasartigen) Geruchs- und/oder Geschmacksnote und zusätzlich einer, zweier oder mehrerer Geruchs- und/oder Geschmacksnoten ausgewählt aus der Gruppe bestehend aus den Noten krautig, frisch, blumig, holzig, süß, erdig, fettig, metallisch und balsamisch.

Die Tatsache, dass die erfindungsgemäßen bzw. erfindungsgemäß einzusetzenden Verbindungen der Formel (I), insbesondere die im Rahmen dieses Textes als bevorzugt bzw. besonders bevorzugt gekennzeichneten Verbindungen der Formel (I), einen sehr komplexen und vielfältigen Geruchs- und Geschmackseindruck, der sonst meist nur von Mischungen mehrerer Komponenten (wie beispielsweise etherischen Ölen, oder Gewürzmischungen) erreicht werden kann.

Über ihre primären, nämlich geruchlichen, Eigenschaften hinaus verfügen die Verbindungen der Formel (I) zusätzlich über positive Sekundäreigenschaften, insbesondere ein gutes Haftungsvermögen und eine hohe Substantivität im Vergleich zu Riechstoffen mit ähnlichen Geruchseigenschaften, sowie eine hohe Stabilität in bestimmten Medien und Zubereitungen und eine hohe Ausgiebigkeit.

Die erfindungsgemäßen bzw. erfindungsgemäß einzusetzenden Verbindungen der Formel (I) weisen insbesondere in weitgehend neutralen, insbesondere jedoch in alkalischen und/oder in oxidierenden Medien eine hohe, ganz hervorragende Stabilität auf. Insbesondere wegen dieser Eigenschaften eignen sich die Verbindungen der Formel (I) in ausgezeichnet für die Verwendung als Riech- und/oder Aromastoffe, und zwar insbesondere, wenn sie in parfümierten oder aromatisierten Artikeln oder Zubereitungen eingesetzt werden, die einen pH-Wert von 5,5 oder größer besitzen, vorzugsweise von größer oder gleich 6, bevorzugt von größer 7, weiter bevorzugt von größer 7,5, insbesondere bevorzugt von größer 8; ferner in oxidierend wirkenden Zubereitungen, die vorzugsweise eine pH-Wert von größer oder gleich 7 aufweisen, bevorzugt in oxidierend wirkenden Zubereitungen mit einem pH-Wert von größer oder gleich 8. Die angegebenen pH-Werte beziehen sich dabei jeweils auf bei 25°C gemessenen Werte.

Im Zusammenhang mit der bevorzugten Verwendung zum Vermitteln, Modifizieren und/oder Verstärken einer Geruchs- und/oder Geschmacksnote steht auch die Erkenntnis, dass eine oder mehrere Verbindungen der Formel (I) hervorragend als sogenannte Booster (Verstärker; Enhancer) fungieren können (siehe auch untenstehende Beispiele 2 und 3), insbesondere der Noten blumig, fruchtig und/oder holzig.

Ein entsprechendes erfindungsgemäßes Verfahren zum Modifizieren und/oder Verstärken (Boosten) eines Geruchs und/oder Geschmacks mit einer, mehreren oder sämtlichen der Noten blumig, fruchtig und/oder holzig umfasst den folgenden Schritt:
- Vermischen eines oder mehrerer Riech- oder Aromastoffe mit einer, mehreren oder sämtlichen der Noten blumig, fruchtig und/oder holzig mit einer Menge an einer oder mehreren Verbindungen der Formel (I) die ausreicht, den Geruchs- und/oder Geschmackseindruck des bzw. der Riech- oder Aromastoffe, die eine oder mehrere der Noten blumig, fruchtig und/oder holzig verursachen, sensorisch zu modifizieren und/oder zu verstärken.

Die erfindungsgemäßen bzw. erfindungsgemäß zu verwendenden Verbindungen der Formel (I) können zudem die Intensität einer Riech- bzw. Aromastoffmischung verstärken und das Gesamtbild der Mischung geruchlich abrunden und können eingesetzt werden, um einer Riech- oder Aromastoffkomposition mehr Fülle, Frische, (Strahl)Kraft, Ausstrahlung, Glanz, Abrundung, Harmonie und/oder Natürlichkeit zu verleihen.

Eine oder mehrere Verbindungen der Formel (I) können insbesondere eingesetzt werden, um einer Riech- oder Aromastoffkomposition Frische, (Aus)Strahlung, Abrundung, Harmonie und/oder Natürlichkeit zu verleihen und/oder vorhandene Geruchs- und/oder Geschmacksnoten zu verstärken (siehe auch untenstehende Beispiele 2 und 3).

Die vorliegende Erfindung betrifft auch die Verwendung einer oder mehrere Verbindungen der Formel (I) zum Versehen von Haaren und/oder Haut oder von textilen Fasern mit einem Duft (hinsichtlich der dabei bevorzugt vermittelten Geruchs- bzw. Geschmacksnoten sei auf die obigen Ausführungen verwiesen), wobei die Verbindung(en) der Formel (I) vorzugsweise in Kombination mit einem Tensid oder einer Tensidmischung eingesetzt wird bzw. werden.

Die vorliegende Erfindung betrifft auch entsprechende Verfahren und (vorzugsweise tensidhaltige) Mischungen. Gemäß einem verwandten Aspekt betrifft die vorliegende Erfindung auch die Verwendung der Verbindungen der Formel (I) oder einer Mischung von Verbindungen der Formel (I) als Mittel zum Erhöhen der Substantivität und/oder Retention einer Riechstoffmischung und/oder als Fixateur.

Bevorzugt ist die Verwendung einer Verbindung der Formel (I), wobei R und R1
- jeweils unabhängig voneinander Wasserstoff oder einen organischen Rest mit 1 bis 15 Kohlenstoffatomen bedeuten, wobei
   (i) unabhängig voneinander einer der Reste R und R1 Wasserstoff und der andere Rest einen organischen Rest mit 1 bis 15 Kohlenstoffatomen bedeutet
      oder
   (ii) unabhängig voneinander sowohl R als auch R1 einen organischen Rest mit
      1 bis 15 Kohlenstoffatomen bedeuten,
   oder
- zusammen mit dem Kohlenstoffatom, an das sie gebunden sind, einen Ring mit 5, 6, 7 oder 8 Ring-Kohlenstoffatomen bilden.

Bevorzugt ist die Verwendung einer Verbindung der Formel (I), wobei R und R1 jeweils unabhängig voneinander Wasserstoff oder einen organischen Rest mit 1 bis 15 Kohlenstoffatomen bedeuten, wobei der, einer oder jeder organische Rest ein
gesättigter oder ungesättigter Rest ist,
und/oder
ein aromatischer oder aliphatischer Rest ist,
und/oder
ein alicyclischer oder nicht-cyclischer Rest ist,
und/oder
ein verzweigter oder unverzweigter Rest ist,
und/oder
der, einer oder jeder organische Rest 0, 1, 2 oder 3 Sauerstoff-Atome enthält.

Bevorzugt ist die Verwendung einer Verbindung der Formel (I), wobei R oder R1 oder beide unabhängig voneinander einen organischen, aliphatischen Rest mit 1 bis 15 Kohlenstoffatomen bedeuten, wobei vorzugsweise der jeweilige aliphatische Rest ausgewählt ist aus der Gruppe bestehend aus:
Methyl, Ethyl, n-Propyl, iso-Propyl, n-Butyl, sec-Butyl, iso-Butyl, tert-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
But-3-enyl, 2,6-Dimethylhept-5-enyl, 3,5-Dimethylhex-4-enyl, 4-Mehtylpent-3-enyl, 2,6-Dimethylhepta-1,5-dienyl und 2-Methylpropenyl.

Von den Verbindungen der Formel (I), in denen R oder R1 oder beide unabhängig voneinander einen organischen, aliphatischen Rest mit 1 bis 15 Kohlenstoffatomen bedeuten, ist eine bevorzugte Verbindung ausgewählt aus der Gruppe bestehend aus den Verbindungen der Formeln 3, 14, 15, 16, 17, 18, 19, 20, 21, 22 und 23:

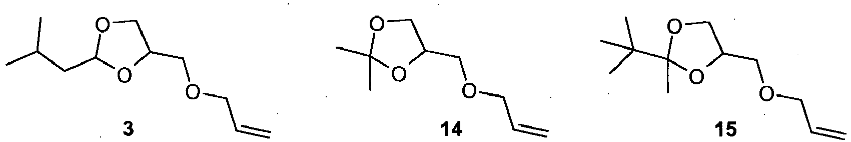

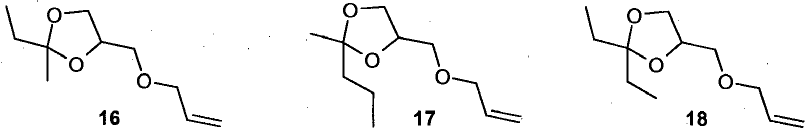

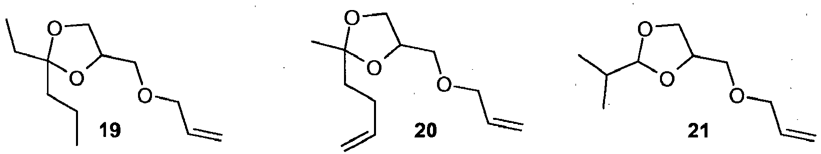

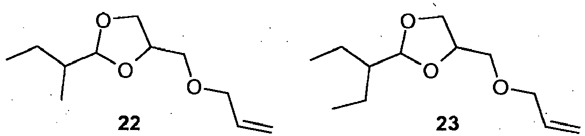

Ebenfalls bevorzugt ist die Verwendung einer Verbindung der Formel (I), wobei R und R1 zusammen mit dem Kohlenstoffatom, an das sie gebunden sind, einen Ring bilden, der
- eine oder zwei Doppelbindungen umfasst,
   und/oder
- 1, 2 oder 3 Sauerstoffatome enthält,
   und/oder
- im Bereich der Reste R und R1 unsubstituiert oder substituiert ist mit einer oder mehreren verzweigten oder unverzweigten Alkylgruppen, Alkenylgruppen, Cycloalkylgruppen, Cycloalkenylgruppen, Arylgruppen, Arylalkylgruppen, Alkoxyalkylgruppen oder Alkoxyarylgruppen,
   und/oder
- zusammen mit seinen gegebenenfalls vorhandenen Substituenten 5 bis 30 Kohlenstoff-Atome, bevorzugt 5 bis 15 Kohlenstoff-Atome, umfasst.

Dabei bevorzugt ist die Verwendung einer Verbindung der Formel (I), wobei R und R1 zusammen mit dem Kohlenstoffatom, an das sie gebunden sind, einen Ring bilden, wobei der Ring ein Carbocyclus oder ein Heterocyclus ist, weiter bevorzugt ein aliphatischer Carbocyclus oder Heterocyclus ist.

Dabei weiter bevorzugt ist die Verwendung einer Verbindung der Formel (I), wobei R und R1 zusammen mit dem Kohlenstoffatom, an das sie gebunden sind, einen Ring bilden, der im Bereich der Reste R und R1 substituiert ist mit einer oder mehreren Substituenten ausgewählt aus der Gruppe bestehend aus: Methyl, Ethyl, n-Propyl, iso-Propyl, n-Butyl, sec-Butyl, iso-Butyl, tert-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, But-3-enyl, 2,6-Dimethylhept-5-enyl, 3,5-Dimethylhex-4-enyl, 4-Mehtylpent-3-enyl, 2,6-Dimethylhepta-1,5-dienyl, 2-Methylpropenyl.

Von den Verbindungen der Formel (I), in denen R und R1 zusammen mit dem Kohlenstoffatom, an das sie gebunden sind, einen Ring bilden, ist eine bevorzugte Verbindung ausgewählt aus der Gruppe bestehend aus den Verbindungen der Formeln 2, 4, 8, 1, 9, 10, 11, 7, 5, 6, 12 und 13:

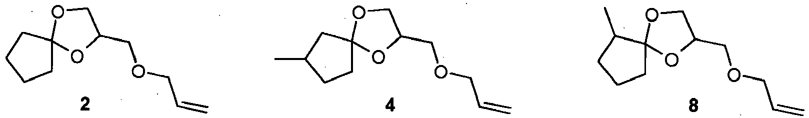

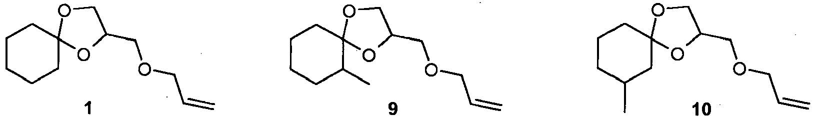

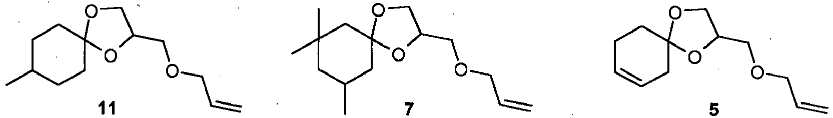

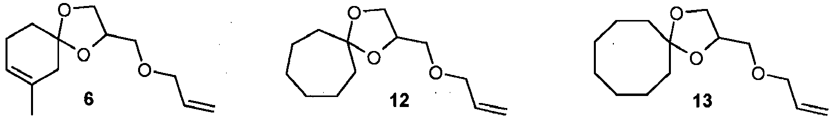

Erfindungsgemäß bevorzugte Verbindungen der Formel (I) umfassen insgesamt höchstens 20 Kohlenstoff-Atome, bevorzugt höchstens 18 Kohlenstoff-Atome, weiter bevorzugt höchstens 16 Kohlenstoff-Atome, insbesondere bevorzugt höchstens 15 Kohlenstoff-Atome. In einer regelmäßig bevorzugten Ausgestaltung umfasst eine bevorzugte Verbindung der Formel (I) insgesamt höchstens 14 Kohlenstoff-Atome.

Die nachfolgende Tabelle 1 enthält die sensorischen, insbesondere die geruchlichen, Beschreibungen von einigen erfindungsgemäßen bzw. erfindungsgemäß einzusetzenden Verbindungen der Formel (I).

Sofern in Rahmen dieses Textes versehentlich eine Unstimmigkeit zwischen dem chemischen Namen und der dargestellten Strukturformel der Verbindungen der Formeln (I) auftreten sollte, gilt die dargestellte Strukturformel.

**Tabelle 1:**

| **Nr.:** | **Verbindung** | **Struktur** | **Sensorische Beschreibung** |
|---|---|---|---|
| 1 | 2-Allyloxymethyl-1,4-dioxa-spiro[4.5]decan | 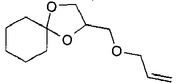 | Stark, sehr stark strahlend, galbanumartig, fruchtig, sehr natürlicher Geruchseindruck |
| 2 | 2-Allyloxymethyl-1,4-dioxa-spiro[4.4]nonan | 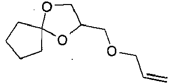 | stark strahlend, galbanumartig, fruchtig, Jasmin, pilzig |
| 3 | 4-Allyloxymethyl-2-isobutyl-[1,3]dioxolan | 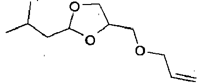 | stark, galbanumartig, fruchtig |
| 4 | 2-Allyloxymethyl-7-methyl-1,4-dioxa-spiro[4.4]nonan | 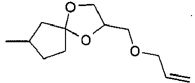 | galbanumartig, fruchtig, Ananas, erinnert an Dynascone |
| 5 | 2-Allyloxymethyl-1,4-dioxa-spiro [4.5]dec-7-en | 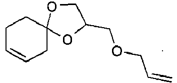 | grün, galbanumartig, fruchtig, Ananas, erinnert an Dynascone |
| 6 | 2-Allyloxymethyl-7-methyl-1,4-dioxa-spiro [4.5]dec-7-en | 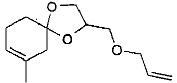 | fruchtig, Ananas, Marzipan |
| 7 | 2-Allyloxymethyl-7,7,9-trimethyl-1,4-dioxa-spiro[4.5]decan | 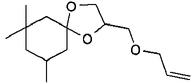 | süß, krautig, holzig |
| 8 | 2-Allyloxymethyl-6-methyl-1,4-dioxa-spiro[4.4]nonan | 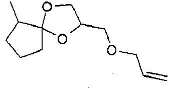 | rosig, fruchtig, Galbanum |
| 9 | 2-Allyloxymethyl-6-methyl-1,4-dioxa-spiro[4.5]decan | 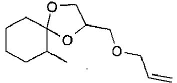 | grün, frisch, Galbanum |
| 10 | 2-Allyloxymethyl-7-methyl-1,4-dioxa-spiro[4.5]decan | 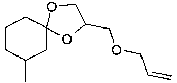 | grün, krautig, medizinisch |
| 11 | 2-Allyloxymethyl-8-methyl-1,4-dioxa-spiro[4.5]decan | 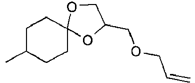 | grün, fettig, fruchtig, krautig |
| 12 | 2-Allyloxymethyl-1,4-dioxa-spiro[4.6]undecan | 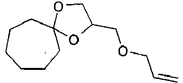 | fruchtig, frisch, Galbanum |
| 13 | 2-Allyloxymethyl-1,4-dioxa-spiro[4.7]dodecan | 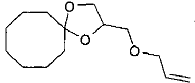 | fruchtig, frisch |
| 14 | 4-Allyloxymethyl-2,2-dimethyl-[1,3]dioxolane | 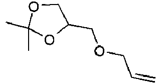 | grün, muffig, fruchtig, |
| 15 | 4-Allyloxymethyl-2-tert-butyl-2-methyl-[1,3]dioxolan | 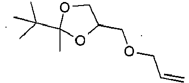 | frisch, grün, krautig, holzig |
| 16 | 4-Allyloxymethyl-2-ethyl-2-methyl-[1,3]dioxolan | 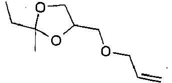 | grün, muffig, erdig, süß, animalisch, |
| 17 | 4-Allyloxymethyl-2-methyl-2-propyl-[1,3]dioxolan | 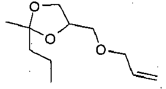 | grün, metallisch, Ananas Galbanum |
| 18 | 4-Allyloxymethyl-2,2-diethyl-[1,3] dioxolan | 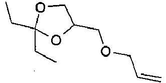 | fettig, fruchtig, iridonartig |
| 19 | 4-Allyloxymethyl-2-ethyl-2-propyl-[ 1,3]dioxolan | 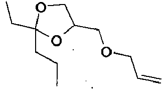 | stark, grün, fruchtig, Sternfrucht |
| 20 | 4-Allyloxymethyl-2-but-3-enyl-2-met hyl-[1,3]dioxolan | 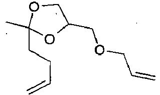 | grün, muffig, Seetang |
| 21 | 4-Allyloxymethyl-2-isopropyl-[1,3] dioxolan | 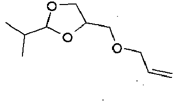 | fruchtig, schokoladig, erdig, Mokka |
| 22 | 4-Allyloxymethyl-2-sec-butyl-[1,3] dioxolan | 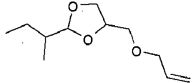 | fruchtig, grün, süß |
| 23 | 4-Allyloxymethyl-2-(1-ethyl-propyl) -[1,3]dioxolan | 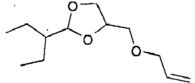 | fruchtig, grün, süß |

Die nachfolgende Tabelle enthält z. B. die sensorische, insbesondere die geschmackliche, Beschreibung der erfindungsgemäß einzusetzenden Verbindung 2-Allyloxymethyl-1,4-dioxa-spiro[4.5]decan.

| **Dosierung** | **Verbindung** | **Struktur** | **Sensorische Be-schreibung** |
|---|---|---|---|
| 3 ppm in 5 gew.-%iger Zuckerlösung | 2-Allyloxymethyl-1,4-dioxa-spiro[4.5]decan | 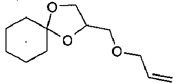 | Voll, minzig, süß, Ananas, überreif schöner Geschmack nach Ananas |

Es wurde in eigenen Untersuchungen gefunden, dass die aus Isovaleraldehyd erhältlichen Acetale der Formel (I) sowie die aus Cyclopentanonen bzw. Cyclohexanonen erhältlichen Ketale der Formel (I) (d.h. Ketale von Cyclopentanon und von substituierten Cyclopentanonen bzw. Ketale von Cyclohexanon und von substituierten Cyclohexanonen) sensorisch besonders wertvoll sind, insbesondere die Ketale von Cyclopentanonen und Cyclohexanonen.

Neben 4-Allyloxymethyl-2-isobutyl-[1,3]dioxolan (Verbindung 3 aus Tabelle 1) sind insbesondere die beiden Verbindungen 2-Allyloxymethyl-1,4-dioxa-spiro[4.5]decan (Verbindung 1 aus Tabelle 1) und 2-Allyloxymethyl-1,4-dioxa-spiro[4.4]nonan (Verbindung 2 aus Tabelle 1) sensorisch interessant.

Die Geruchseigenschaften der erfindungsgemäß besonders bevorzugten bzw. besonders bevorzugten einzusetzenden Ketale können wie folgt beschrieben werden: sehr stark strahlend, galbanumartig, fruchtig, sehr natürlicher Geruchseindruck. Insbesondere können die Ketale zum Vermitteln und/oder Verstärken einer entsprechenden Geruchs- und/oder Geschmacksnote eingesetzt werden.

Einige Verbindungen der Formel (I) sind aus der Literatur bereits bekannt. Allerdings werden im Stand der Technik deren sensorische Eigenschaften und deren Eignung als Riech- oder Aromastoff nicht beschrieben.

Die Herstellung der erfindungsgemäßen bzw. erfindungsgemäß zu verwendenden Verbindungen der Formel (I) kann mittels an sich bekannter Reaktionen und Verfahren erfolgen. Zum Beispiel können entsprechende Diole mit entsprechenden Aldehyden oder Ketonen, vorzugsweise unter sauer katalysierter Wasserabspaltung, zu den entsprechenden Acetalen oder Ketalen der Formel (I) umgesetzt werden. Dieser Reaktionstyp ist in dem nachfolgenden Schema exemplarisch ausgehend von einem kommerziell verfügbaren Diol und Cyclohexanon bzw. Cyclopentanon dargestellt.

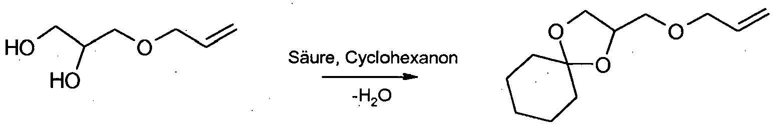

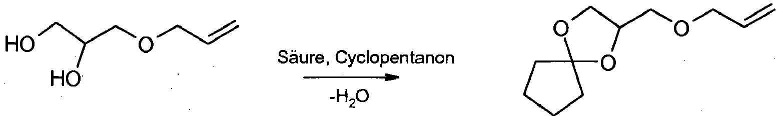

Die erfindungsgemäßen bzw. erfindungsgemäß einzusetzenden Verbindungen der Formel (I) können auf diesem Wege auch in optisch aktiver Form synthetisiert werden (die dafür eingesetzten Edukte sind regelmäßig auch kommerziell erhältlich).

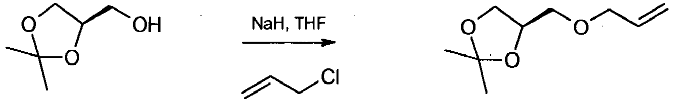

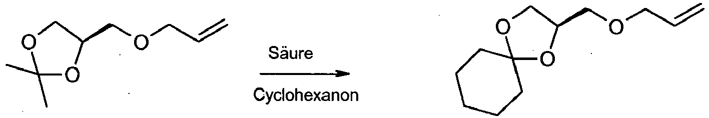

Die Umsetzung von ((R)-2,2-Dimethyl-[1,3]dioxolan-4-yl)-methanol mit Allylchlorid und Natriumhydrid in THF erfolgte unter Erhitzen unter Rückfluss. Die anschließende Umketalisierung der so erhaltenen Verbindung mit Cyclohexanon erfolgte unter Erhitzen unter Rückfluss in Gegenwart einer Säure.

Die Umsetzung ((S)-2,2-Dimethyl-[1,3]dioxolan-4-yl)-methanol wurde analog durchgeführt, wobei das folgende Ketal erhalten wurde:

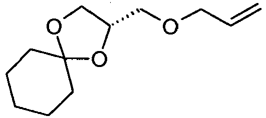

Es wurde in eigenen Untersuchungen gefunden, dass (R)-2-Allyloxymethyl-1,4-dioxa-spiro[4.5]decan geruchlich stärker als ist als (S)-2-Allyloxymethyl-1,4-dioxa-spiro[4.5]decan.

Die erfindungsgemäßen bzw. erfindungsgemäß einzusetzenden Verbindungen der Formel (I) werden im Rahmen der erfindungsgemäßen Verwendung üblicherweise in einer sensorisch wirksamen Menge eingesetzt, d.h. in einer Gesamtmenge, in der sie eine sensorische Wirkung entfalten. Vorzugsweise werden die erfindungsgemäßen bzw. erfindungsgemäß einzusetzenden Verbindungen der Formel (I) zusammen mit anderen Riech- oder Aromastoffen eingesetzt. Solche Riech- oder Aromastoffkompositionen können in üblicher Weise hergestellt werden, beispielsweise durch einfaches Vermischen oder Homogenisieren der Bestandteile. Bei diesen weiteren Riech- oder Aromastoffen kann es sich um beliebige weitere Riech- oder Aromastoffe handeln. Das Gewichtsverhältnis der Gesamtmenge an Verbindungen der Formel (I) zu der Gesamtmenge an weiteren Riech- oder Aromastoffen liegt vorzugsweise im Bereich von 1: 1000 bis 1: 0,5.

Die vorstehend mit Blick auf die erfindungsgemäßen Verwendungen beschriebenen bevorzugten Ausgestaltungen der Erfindung gelten entsprechend auch für erfindungsgemäße Riech- oder Aromastoffkompositionen sowie erfindungsgemäße parfümierte bzw. aromatisierte Artikel, insbesondere die Angaben zu bevorzugten Gewichtsverhältnissen.

Die vorliegende Erfindung betrifft auch bestimmte Verbindungen der Formel (I) 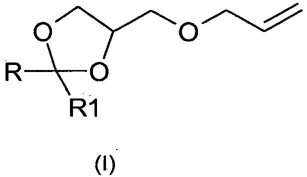 wobei
- unabhängig voneinander einer der Reste R und R1 Wasserstoff und der andere Rest Prop-2-en-1-yl, n-Propyl oder iso-Propyl oder einen aliphatischen, nichtcyclischen Rest mit 4 bis 15 Kohlenstoffatomen bedeutet, der gesättigt oder ungesättigt ist und gleichzeitig verzweigt oder unverzweigt ist,
   oder
- unabhängig voneinander einer der Reste R und R1 Methyl und der andere Rest einen aliphatischen, nicht-cyclischen, ungesättigten Rest mit 2 bis 15 Kohlenstoffatomen bedeutet, der verzweigt oder unverzweigt ist,
   oder
- unabhängig voneinander sowohl R als auch R1 einen aliphatischen, nichtcyclischen Rest mit 2 bis 15 Kohlenstoffatomen bedeuten, der jeweils gesättigt oder ungesättigt ist und gleichzeitig jeweils verzweigt oder unverzweigt ist,
   oder
- zusammen mit dem Kohlenstoffatom, an das sie gebunden sind, einen Ring mit 5, 7 oder 8 Ring-Kohlenstoffatomen bilden,
   oder
- zusammen mit dem Kohlenstoffatom, an das sie gebunden sind, einen Ring mit 6 Ring-Kohlenstoffatomen bilden, wobei der Ring im Bereich der Reste R und R1 substituiert und/oder ungesättigt ist,
   oder
- zusammen mit dem Kohlenstoffatom, an das sie gebunden sind, entweder
   (i) einen Ring mit genau einem Ring-Sauerstoffatom bilden, wobei der Ring (a) 6 Ring-Kohlenstoffatome besitzt und das Sauerstoffatom nicht an das Kohlenstoffatom gebunden ist, an das R und R1 gebunden sind oder (b) 5, 7 oder 8 Ring-Kohlenstoffatome besitzt oder
   (ii) einen Ring mit mehreren Ring-Sauerstoffatomen bilden.

Erfindungsgemäß bevorzugt ist eine Verbindung, wobei R und R1 zusammen mit dem Kohlenstoffatom, an das sie gebunden sind, einen Ring bilden, der
insgesamt 5 bis 16 Kohlenstoff-Atome umfasst
und/oder
eine oder zwei Doppelbindungen umfasst
und/oder
1, 2 oder 3 Sauerstoffatome enthält
und/oder
im Bereich der Reste R und R1 unsubstituiert oder substituiert ist mit einer oder mehreren verzweigten oder unverzweigten Alkylgruppen, Alkenylgruppen, Cycloalkylgruppen, Cycloalkenylgruppen, Arylgruppen, Arylalkylgruppen, Alkoxyalkylgruppen oder Alkoxyarylgruppen.

Dabei bevorzugt ist eine Verbindung der Formel (I), wobei R und R1 zusammen mit dem Kohlenstoffatom, an das sie gebunden sind, einen Ring bilden, wobei der Ring ein Carbocyclus oder ein Heterocyclus ist, weiter bevorzugt ein aliphatischer Carbocyclus oder Heterocyclus ist.

Ebenfalls bevorzugt ist eine erfindungsgemäße Verbindung der Formel (I), wobei R und R1 zusammen mit dem Kohlenstoffatom, an das sie gebunden sind, einen Ring bilden, der im Bereich der Reste R und R1 substituiert ist mit einer oder mehreren Substituenten ausgewählt aus der Gruppe bestehend aus: Methyl, Ethyl, n-Propyl, iso-Propyl, n-Butyl, sec-Butyl, iso-Butyl, tert-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, But-3-enyl, 2,6-Dimethylhept-5-enyl, 3,5-Dimethylhex-4-enyl, 4-Mehtylpent-3-enyl, 2,6-Dimethylhepta-1,5-dienyl, 2-Methylpropenyl.

Besonders bevorzugte Verbindungen der Formel (I) sind ausgewählt aus der Gruppe bestehend aus den Verbindungen mit den Formeln 2, 3, 4, 5, 6, 7, 8, 9, 10, 11, 12, 13, 18, 19, 20, 21, 22 und 23:

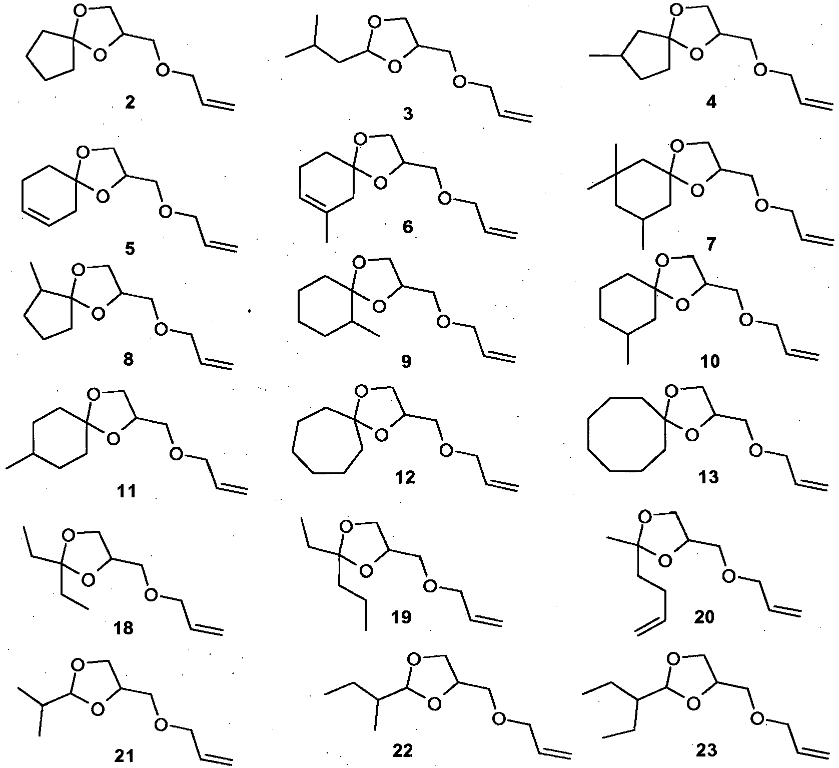

Die vorliegende Erfindung betrifft auch parfümierte oder aromatisierte Artikel, umfassend
- eine oder mehrere Verbindungen der Formel (I) wie oben definiert, vorzugsweise in einer der vorstehend als bevorzugt angegebenen Ausgestaltungen, vorzugsweise in einer sensorisch wirksamen Menge,
- einen, zwei, drei, vier, fünf, sechs, sieben, acht, neun, zehn oder mehr weitere Riech- oder Aromastoffe, wobei vorzugsweise einer, mehrere oder sämtliche der weiteren Riech- oder Aromastoffe einen holzigen, fruchtigen und/oder blumigen Geruch und/oder Geschmack vermitteln,
- einen oder mehrere weitere Zusatz-, Hilfs- und/oder Wirkstoffe, vorzugsweise zwei, drei, vier, fünf oder mehr Zusatz-, Hilfs- und/oder Wirkstoffe.

Es versteht sich dabei, dass die weiteren Zusatz-, Hilfs- und/oder Wirkstoffe keine Riech- oder Aromastoffe sind.

Zusatz-, Hilfs- und/oder Wirkstoffe, welche ein erfindungsgemäßer parfümierter und/oder aromatisierter Artikel zusätzlich zu einer oder mehreren Verbindungen der Formel (I) bzw. zusätzlich zu einer erfindungsgemäßen Riech- oder Aromastoffkomposition (wie nachfolgend definiert) enthalten kann, sind vorzugsweise ausgewählt aus der Gruppe bestehend aus:
Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildner, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, (weitere) Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-betadicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, fette Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Farbstoffe und farbschützende Mittel sowie Pigmente, vorzugsweise die in WO 2005/123101 genannten, Antikorrosiva, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, haarwachstumsmodulierende Mittel (haarwachstumsfördend oder haarwachstumshemmend), vorzugsweise die in EP 2168570 und EP 2193785 genannten oder Silikone und Silikonderivate, vorzugsweise die in WO 2008/046676 genannten.

Die vorliegende Erfindung betrifft vorzugsweise parfümierte oder aromatisierte Artikel, umfassend
- eine oder mehrere Verbindungen der Formel (I) wie oben definiert, vorzugsweise in einer der vorstehend als bevorzugt angegebenen Ausgestaltungen, in einer sensorisch wirksamen Menge, die ausreicht, eine oder mehrere der Geruchs- oder Geschmacksnoten grün, krautig, frisch, fruchtig, blumig, holzig, süß, erdig, fettig, metallisch und balsamisch, zu vermitteln, zu modifizieren und/oder zu verstärken, bevorzugt zu vermitteln und/oder zu verstärken,
- einen, zwei, drei, vier, fünf, sechs, sieben, acht, neun, zehn oder mehr weitere Riech- oder Aromastoffe, wobei vorzugsweise einer, zwei, drei oder mehrere der weiteren Riech- oder Aromastoffe einen holzigen, fruchtigen und/oder blumigen Geruch und/oder Geschmack vermitteln,
- zwei, drei, vier, fünf oder mehr Zusatz-, Hilfs- und/oder Wirkstoffe, wobei bevorzugt ein, zwei, drei oder mehr Zusatz-, Hilfs- und/oder Wirkstoffe ausgewählt sind aus der Gruppe bestehend aus Konservierungsmitteln, anorganischen Salzen, Chelatbildnern, Tensiden, haut- und/oder haarpflegenden Mitteln, Enzymen Emulgatoren, Fetten, fetten Ölen, Wachsen, Fettalkoholen, Silikonen, Silikonderivaten und Wasser.

In einer bevorzugten Ausgestaltung ist ein erfindungsgemäß parfümierter oder aromatisierter Artikel ausgewählt aus der Gruppe bestehend aus Wasch- und Reinigungsmitteln, Hygiene- oder Pflegeprodukten, insbesondere im Bereich der Körper- und Haarpflege, der Kosmetik und des Haushalts.

Die Verbindungen der Formel (I) eignen sich wegen ihrer olfaktorischen Eigenschaften vorzüglich für den Einsatz in Riech- und Aromastoffkompositionen. Eine oder mehrere Verbindungen der Formel (I) können mit einer Vielzahl weiterer Riech- oder Aromastoffe kombiniert und in zahlreichen unterschiedlichen Produkten und Artikeln verwendet werden. Besonders vorteilhaft lassen sich die Verbindungen der Formel (I) mit anderen Riech- oder Aromastoffen in verschiedenen, unterschiedlichen Mengenverhältnissen zu erfindungsgemäßen Riech- oder Aromastoffkompositionen kombinieren.

In einem weiteren Aspekt betrifft die vorliegende Erfindung daher eine Riech- oder Aromastoffkomposition umfassend:
a) eine oder mehrere Verbindungen der Formel (I) wie oben definiert sowie
b) einen oder mehrere, vorzugsweise zwei, drei, vier, fünf, sechs, sieben, acht, neun, zehn oder mehr, weitere Riech- oder Aromastoffe,
   (i) wobei der oder die weiteren Riech- oder Aromastoffe einen holzigen, fruchtigen und/oder blumigen Geruch und/oder Geschmack vermitteln,
      oder
   (ii) wobei der oder die weiteren Riech- oder Aromastoffe einen anderen Geruch und/oder Geschmack vermitteln.

In einer bevorzugten erfindungsgemäßen Riech- oder Aromastoffkomposition liegt das Gewichtsverhältnis der Gesamtmenge an Verbindungen der Formel (I), vorzugsweise in einer der vorstehend als bevorzugt angegebenen Ausgestaltungen, zu der Gesamtmenge an weiteren Riech- oder Aromastoffen im Bereich von 1 : 1000 bis 1 : 0,5.

Da eine erfindungsgemäße Riech- oder Aromastoffkomposition zur Parfümierung oder Aromatisierung von Artikeln geeignet ist, betrifft die Erfindung in einem weiteren Aspekt einen parfümierten oder aromatisierten Artikel, umfassend
- eine erfindungsgemäße Riech- oder Aromastoffkomposition, vorzugsweise in einer der als bevorzugt gekennzeichneten Ausgestaltungen, vorzugsweise in einer sensorisch wirksamen Menge,
   und
- einen oder mehrere weitere Zusatz-, Hilfs- und/oder Wirkstoffe, vorzugsweise zwei, drei, vier, fünf oder mehr Zusatz-, Hilfs- und/oder Wirkstoffe, vorzugsweise zwei, drei, vier, fünf oder mehr der oben als bevorzugt angegebenen Zusatz-, Hilfs- und/oder Wirkstoffe.

Insbesondere durch Kombination der Verbindungen der Formel (I) wie oben definiert, vorzugsweise in einer der als bevorzugt angegebenen Ausgestaltungen, mit einem oder mehren weiteren Riech- oder Aromastoffen (vorzugsweise mit holzigem, fruchtigem und/oder blumigem Geruch oder Geschmack) lassen sich interessante neue Riech- oder Aromastoffkompositionen herstellen. Auf diese Weise lassen sich Mischungen mit besonders interessanten, natürlichen, neuen und originellen Noten kreieren. Riechstoffe, die als Komponente b) einer erfindungsgemäßem Riech- oder Aromastoffkomposition geeignet sind, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001.

Im Einzelnen seien genannt: Extrakte aus natürlichen Rohstoffen wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernnadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercapto hexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;
der acyclischen Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Citronellal;; 7-Methoxy-3,7-dimethyloctanal; 2,6,10-Trimethyl-9-undecenal; Geranylaceton; sowie die Dimethyl- und Diethylacetale von Geranial, Neral,
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethyl-ionon; alpha-Iron; beta-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methano-naphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-tert.-Butylcyclohexanol; 3,3,5-Trimethylcyclohexanol; 3-lsocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; . Cedrylmethylether; Cyclododecylmethylether;1,1-Dimethoxycyclododecan; (Ethoxymethoxy)cyclo-dodecan; alpha-Cedrenepoxid; 3a,6,6,9a-Tetramethyldodecahydro-naphtho[2,1-b]furan; 3a-Ethyl-6,6,9a-trimethyldodecahydronaphtho[2,1b]furan; 1,5,9-Tri-methyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1-Ethoxyvinyl)-3,3,5,5-tetramethylcyclohexanon; 4-tert.-Pentylcyclo-hexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2-Methyl-4-(2,2,6-trimethyl-cyclohexen-1-yl)-2-butenal; 4-(4-Hydroxy-4-methylpenfyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentyl-cyclo-hexyl-acetat; 3,3,5-Trimethylcyclohexylacetat; Decahydro-2-naphthylacetat;2-Cyclo-pentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5, bzw. 6-inden-ylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der araliphatischen Alkohole wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenyl-propanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha
,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethyl-isoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetra-hydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-tert.-Butylphenyl)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenz-aldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylaceto-phenon; 4-Phenyl-2-butanon; 4-(4-Hydroxyphenyl)-2-butanon; 1-(2-Naphtha-lenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-di-methyl-4-indanylmethylketon; 1-[2,3-dihydro-1,1,2,6-tetramethyl-3-(1-methylethyl) -1 H-5-indenyl]ethanon; 5',6',7',8'-Tetrahydro-3',5',5',6',8',8'-hexamethyl-2-aceto-naphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butyl-phenyl)propanal oder 2,4-Dimethyl-3-cyclohexencarbaldehyd; 6-Isopropyl-chinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; 1,16-Hexadeca-nolid; 9-Hexadecen-1,16-olid; 10-Oxa-1,16-hexadecanolid; 11-Oxa-1,16-hexa-decanolid; 12-Oxa-1,16-hexadecanolid; Ethylen-1,12-dodecandioat; Ethylen-1,13-tridecandioat; 2,3-Dihydrocumarin; Octahydrocumarin.

In einer bevorzugten Ausgestaltung einer erfindungsgemäßen Riech- oder Aromastoffkomposition sind die erfindungsgemäßen bzw. erfindungsgemäß zu verwendenden Verbindungen der Formel (I) vorzugsweise mit einem oder mehreren, besonders bevorzugt mit zwei, drei, vier, fünf oder mehr, blumigen und/oder fruchtigen weiteren Riech- und/oder Aromastoffen kombiniert.

Entsprechend betrifft die vorliegende Erfindung auch eine Riechstoff- oder Aromastoffkomposition, die einen, zwei, drei, vier, fünf oder mehrere Riech- und/oder Aromastoffe umfasst, die eine blumige und/oder fruchtige Geruchs- oder Geschmacksnote vermitteln.

Dabei wird durch die erfindungsgemäße bzw. erfindungsgemäß zu verwendende Verbindung der Formel (I) vorteilhafterweise (zumindest teilweise) eine geruchliche Verstärkung der blumigen Riech- bzw. Aromastoffe erreicht.

Blumige Riech- oder Aromastoffe, mit denen erfindungsgemäßen bzw. erfindungsgemäß zu verwendenden Verbindungen der Formel (I) (insbesondere in erfindungsgemäßen Riech- oder Aromastofflcompositionen) vorteilhaft kombiniert werden können, werden vorzugsweise ausgewählt aus der Gruppe bestehend aus:
Hydroxycitronellal, Methoxycitronellal, Cyclamenaldehyd [2-Methyl-3-(4-isopropylphenyl)propanal], 1-(4-Isopropyl-cyclohexyl)ethanol (Mugetanol^{®}), 4-tert.-Butyl-α-methyldihydrozimtaldehyd (Lilial^{®}), cis-Hexahydrocuminylalkohol (Mayol^{®}), 3-[4-(1,1-Dimethylethyl)phenyl]propanal (Bourgeonal^{®}), 2,2-Dimethyl-3-(3-methylphenyl)propanol (Majantol^{®}), 3-Methyl-3-(3-methylbenzyl)-butan-2-ol, 2-Isobutyl-4-methyltetrahydro-2H-pyran-4-ol (Florosa^{®}), 2-Methyl-3-(3,4-methylendioxyphenyl)propanal (Heliofolal^{®}), 4-(4-Hydroxy-4-methylpentyl)-3-cyclohexencarbaldehyd (Lyral^{®}), 4-(Octahydro-4,7-methano-5H-inden-5-yliden-butanal (Dupical^{®}), Vernaldehyd, 4-(4-Methy)-3-penten-1-yl)-3-cyclohexencarbaldehyd (Vertomugal^{®}), Octahydro-5-(4-methoxybutyliden)-4,7-methano-1H-inden (Mugoflor^{®}), 2,6-Dimethyl-2-heptanol (Freesiol^{®}), 1-Ethyl-1-methyl-3-phenylpropanol (Phemec^{®}), 2,2-Dimethyl-3-phenyl-1-propanol (Muguet alcohol), Profarnesol, Dihydrofarnesol, Farnesol, Nerolidol, Hydroxycitronellaldimethylacetal, Hexylbenzoat, Geraniol Nerol, Linalool, Tetrahydrogeraniol, Tetrahydrolinalool, Ethyllinalool, Geranyltiglinat, Phenethylalkohol (2-Phenylethylalkohol), Citronellol, Rosenoxid, 2-Methyl-5-phenylpentanol (Rosaphen), 3-Methyl-5-phenylpentanol (Phenoxanol), Methyldihydrojasmonat (Hedion^{®}, Hedione^{®} high cis), 2-Heptylcyclopentanon (Projasmon P), cis-Jasmon, Dihydrojasmon, Zimtalkohol (3-Phenyl-2-propen-1-ol), Dihydrozimtalkohol (3-Phenylpropanol), 2-Methyl-4-phenyl-1,3-dioxolan (Jacinthaflor^{®}) und Dihydromyrcenol (2,6-Dimethyl-7-octen-2-ol).

Zudem sind erfindungsgemäße bzw. erfindungsgemäß zu verwendende Verbindungen der Formel (I) vorteilhafterweise dazu geeignet, fruchtige Riech- oder Aromastoffe, insbesondere fruchtige Riechstoffe, hinsichtlich ihres Geruchs zu verstärken.

Fruchtige Riech- oder Aromastoffe, mit denen die erfindungsgemäßen bzw. erfindungsgemäß zu verwendenden Verbindungen der Formel (I) vorteilhaft kombiniert werden können, und die demnach besonders bevorzugte (weitere) Riech- oder Aromastoffe einer erfindungsgemäßen Riech- oder Aromastoffkompositionen sind, sind vorzugsweise ausgewählt der Gruppe bestehend aus:
2-Methyl-buttersäureethylester, 4-(p-Hydroxyphenyl)-2-butanon, Ethyl-3-methyl-3-phenylglycidat, Buttersäureisoamylester, Essigsäureisoamylester, Essigsäure-n-butylester, Buttersäureethylester, 3-Methyl-buttersäureethylester, n-Hexansäureethylester, n-Hexansäureallylester, Ethyl-2-trans-4-cis-decadienoat, 1,1-Dimethoxy-2,2,5-trimethyl-4-hexan, 2,6-Dimethyl-5-hepten-1-al, gamma-Undecalacton, gamma-Nonalacton, Hexanal, 3Z-Hexenal, n-Decanal, n-Dodecanal, Citral, Vanillin, Ethylvanillin, Maltol, Ethylmaltol und deren Mischungen.

In einer bevorzugten erfindungsgemäßen Riech- oder Aromastoffkomposition liegt die Gesamtmenge an Verbindungen der Formel (I) im Bereich von 0,0001 bis 99,9 Gew.-%, vorzugsweise im Bereich von 0,001 bis 99,5 Gew.-%, besonders bevorzugt im Bereich von 0,01 bis 99 Gew.-%, jeweils bezogen auf die Gesamtmasse der Riech- oder Aromastoffkomposition.

In einer bevorzugten erfindungsgemäßen Riech- oder Aromastoffkomposition liegt die Gesamtmenge an Verbindungen der Formel (I) im Bereich von 0,01 bis 90 Gew.-%, vorzugsweise im Bereich von 0,05 bis 80 Gew.-%, weiter bevorzugt im Bereich von 0,1 bis 70 Gew.-%, besonders bevorzugt im Bereich von 0,25 bis 50 Gew.-%, insbesondere bevorzugt im Bereich von 0,5 bis 40 Gew.-%, am meisten bevorzugt im Bereich von 0,75 bis 25 Gew.-%, jeweils bezogen auf die Gesamtmasse der Riech- oder Aromastoffkom position.

In einer bevorzugten erfindungsgemäßen Riech- oder Aromastoffkomposition reicht die eingesetzte Gesamtmenge an der oder den Verbindungen der Formel (I) wie oben definiert aus, um den Geruch und/oder Geschmack der weiteren Riech- oder Aromastoffe in Richtung grün, krautig, frisch, fruchtig, blumig, holzig, süß, erdig, fettig, metallisch und balsamisch, vorzugsweise in Richtung grün und/oder fruchtig, bevorzugt in Richtung Galbanum, zu modifizieren, zu verstärken und/oder eine oder mehrere der genannten Noten zu vermitteln.

Sofern eine oder mehrere Verbindungen der Formel (I) hauptsächlich eingesetzt wird bzw. werden, um einer Riech- oder Aromastoffkomposition mehr Frische, (Aus)Strahlung, Abrundung, Harmonie und/oder Natürlichkeit zu verleihen und/oder bestimmte (durch weitere Riech- oder Aromastoffe bereits vorhandene) Noten zu verstärken, liegt die Gesamtmenge der Komponente (a) vorzugsweise vergleichsweise niedrig und besonders bevorzugt im Bereich von 0,01 bis 5 Gew.-%, bevorzugt im Bereich von 0,1 bis 2 Gew.-%, bezogen auf die Gesamtmenge der Riech- oder Aromastoffkomposition. Sofern innerhalb der bevorzugten Konzentrationsbereiche eine vergleichsweise niedrige Konzentration gewählt wird, kommt es in Abhängigkeit von den weiteren Komponenten der jeweiligen Komposition in manchen Fällen noch nicht zu der Vermittlung der oben angegebenen Eigengeruchs- oder -geschmacksnoten.

Die Erfindung betrifft in einem weiteren Aspekt die Verwendung einer erfindungsgmäßen Riech- oder Aromastoffkomposition, vorzugsweise in einer der als bevorzugt angegebenen Ausgestaltungen,
(A) als Riech- oder Aromastoffkomposition
   (a)
      (i) mit Galbanum-Note
         und/oder
      (ii) zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, süß, erdig, fettig, metallisch und balsamisch, vorzugsweise zumindest einer der Noten grün und/oder fruchtig,
      und/oder
   (b) (i) mit fruchtiger und/oder mit blumiger Note,
      und/oder
      (ii) zum Vermitteln, Modifizieren und/oder Verstärken einer oder mehrerer Geruchs- und/oder Geschmacksnoten ausgewählt aus der Gruppe bestehend aus den Noten holzig, fruchtig und/oder blumig,
      und/oder
   (c) mit blumiger, fruchtiger, galbanumartiger Note.
   und/oder
(B) als Bestandteil eines parfümierten oder aromatisierten Artikels.

Erfindungsgemäße Riech- oder Aromastoffkompositionen, welche eine oder mehrere Verbindungen der Formel (I) enthalten, können in flüssiger Form, unverdünnt oder mit einem Lösungsmittel verdünnt zur Parfümierung oder Aromatisierung eingesetzt werden. Bevorzugte Lösungsmittel hierfür sind Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat, Triacetin und Diacetin.

Des Weiteren können erfindungsgemäße Riech- oder Aromastoffkompositionen, welche Verbindungen enthalten, an einem Trägerstoff adsorbiert sein, der sowohl für eine feine Verteilung der Riech- oder Aromastoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein.

Die Kombination aus erfindungsgemäßer Komposition und Trägerstoff stellt einen beispielhaften erfindungsgemäßen Artikel dar.

Erfindungsgemäße Riech- oder Aromastoffkompositionen, die Verbindungen enthalten, können auch mikroverkapselt, sprühgetrocknet, als Einschluss-Komplexe oder als Extrusions-Produkte (d. h. erfindungsgemäße Artikel) vorliegen und in dieser Form z. B. einem zu parfümierenden oder aromatisierenden Produkt hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Artikel dar.

Die Mikroverkapselung der erfindungsgemäßen Riech- oder Aromastoffkompositionen zu erfindungsgemäßen Artikeln kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riech- oder Aromastoffkompositionen können beispielsweise durch Sprühtrocknung einer die Riech- oder Aromastoffkomposition enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riech- oder Aromastoffkomposition und Cyclodextrinen oder Harnstoffderivaten in ein geeignetes Lösungsmittel, z.B. Wasser, hergestellt werden. Extrusions-Produkte können durch Verschmelzen der Riech- oder Aromastoffkompositionen mit einem geeigneten wachsartigen Stoff und durch Extrusion mit nachfolgender Erstarrung, gegebenenfalls in einem geeigneten Lösungsmittel, z.B. Isopropanol, erhalten werden.

Verbindungen der Formel (I) und Riech- oder Aromastoffkompositionen, die eine oder mehrere Verbindungen der Formel (I) enthalten, können in konzentrierter Form, in Lösungen oder in oben beschriebener modifizierter Form verwendet werden für die Herstellung von erfindungsgemäßen parfümierten Artikeln wie z. B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Ein erfindungsgemäß bevorzugter parfümierter oder aromatisierter Artikel (wie oben definiert) ist ausgewählt aus der Gruppe bestehend aus

Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

In einem erfindungsgemäß bevorzugten parfümierten oder aromatisierten Artikel liegt die Gesamtmenge an Verbindungen der Formel (I), vorzugsweise in einer der vorstehend als bevorzugt angegebenen Ausgestaltungen, im Bereich von 0,00001 bis 10 Gew.-%, vorzugsweise im Bereich von 0,0001 bis 5 Gew.-%, besonders bevorzugt im Bereich von 0,001 bis 2 Gew.-%, am meisten bevorzugt 0,005 bis 1 Gew.-%, jeweils bezogen auf die Gesamtmasse des parfümierten oder aromatisierten Artikels.

Die Verbindungen der Formel (I) bzw. Mischungen von Verbindungen der Formel (I) können in aromatisierte oder zu aromatisierende Artikel eingearbeitet werden, insbesondere in der Ernährung, der Mundpflege oder dem Genuss dienende Zubereitungen.

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiss, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Nach Einarbeiten der erfindungsgemäßen Verbindungen bzw. der entsprechenden Mischungen sind diese Zubereitungen erfindungsgemäße Zubereitungen (als Beispiel erfindungsgemäßer Artikel).

Erfindungsgemäße Zubereitungen können z. B. als Halbfertigware oder als Würzmischung vorliegen.

Erfindungsgemäße Zubereitungen können insbesondere als Halbfertigware zur Herstellung weiterer der Ernährung oder dem Genuss dienenden Zubereitungen dienen, insbesondere in sprühgetrockneter Form. Erfindungsgemäße Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende erfindungsgemäße Zubereitungen sind insbesondere Mund- und/oder Zahnpflegemittel wie Zahnpasten, Zahngele, Zahnpulver, Mundwässer, Kaugummis und andere Mundpflegemittel.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,9999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,9 Gew.-%, vorzugsweise 5 bis 80 Gew.-%, bezogen auf das Gesamtgewicht der Zubereitung, aufweisen.

Erfindungsgemäße Zubereitungen (als Beispiele erfindungsgemäßer Artikel), enthaltend Verbindungen, werden gemäß einer bevorzugten Ausgestaltung hergestellt, indem Verbindungen als Substanz, als Lösung (z. B. in Ethanol, Wasser oder 1,2-Propylenglycol) oder in Form eines Gemisches mit einem festen oder flüssigen Trägerstoff (z.B. Maltodextrin, Stärke, Silicagel), sonstigen Aromen oder Aromastoffen und gegebenfalls weiteren Hilfsmitteln und/oder Stabilisatoren (z.B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (Halbfertigware) überführt werden.

Die erfindungsgemäßen sprühgetrockneten festen Zubereitungen (als Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 Gew.-% Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen eine oder mehrere Verbindungen der Formel (I) und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine- oder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung werden Verbindungen zunächst mit einem oder mehreren Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße Zubereitung, bei der die Matrix so gewählt ist, dass die Verbindungen der Formel (I) verzögert von der Matrix freigegeben werden, so dass eine langanhaltende Wirkung erzielt wird. Besonders bevorzugt ist insoweit eine Fett-, Wachs-, Polysaccharid- oder Proteinmatrix.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Basis für die Mundpflege dienende Zubereitungen), die eine oder mehrere Verbindungen der Formel (I) enthalten, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für die Mundpflege dienende Zubereitungen), welche eine oder mehrere Verbindungen der Formel (I) enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Ferner betrifft die vorliegende Erfindung ein Verfahren zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, süß, erdig, fettig, metallisch und balsamisch, vorzugsweise zumindest einer der Noten grün und/oder fruchtig, bevorzugt einer Galbanum-Note, mit folgenden Schritten
- Bereitstellen einer oder mehrerer Verbindungen der Formel (I) wie oben definiert, vorzugsweise in einer der vorstehend als bevorzugt angegebenen Ausgestaltungen, oder einer erfindungsgemäßen Riech- oder Aromastoffkomposition wie oben definiert, vorzugsweise in einer der vorstehend als bevorzugt angegebenen Ausgestaltungen,
- Bereitstellen eines Artikels,
- In Kontakt bringen des Artikels mit einer sensorisch wirksamen Menge von der oder den Verbindungen der Formel (I) bzw. von der Riech- oder Aromastoffkomposition wie oben definiert.

Ein entsprechendes bevorzugtes erfindungsgemäßes Verfahren ist dabei ein Verfahren, bei dem nach dem in Kontakt bringen (i) ein parfümierter Artikel resultiert oder (ii) aus dem dann vorliegenden Erzeugnis hergestellt wird, der ausgewählt ist aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

Nachfolgend wird die Erfindung anhand von Beispielen näher erläutert. Sofern nicht anders angegeben, beziehen sich alle Angaben auf das Gewicht.

Verwendete Abkürzungen: DPG: Dipropylenglycol, TEC = Triethylcitrat

### Beispiel 1: Herstellung von 2-Allyloxymethyl-1,4-dioxa-spiro[4.5]decan

In einen 250 ml Rührwerk wurden 66 g Schwefelsäure 55%ig und 50 g Cyclohexan vorgelegt. Anschließend wurde bei Umgebungstemperatur (ca. 23°C) unter Eiskühlung ein Gemisch aus 67 g (0,5mol) Glycerin-1-allylether und 39,2 g (0,4 mol) Cyclohexanon zugetropf und nach beendeter Zugabe weitere 4 h bei Umgebungstemperatur nachgerührt. Die wässrige Phase wurde anschließend abgetrennt, die organische Phase mit Soda-Lösung neutral gewaschen und eingeengt. Das Rohprodukt (78 g) wurde an einer 30 cm Vigreux-Kolonne im Vakuum fraktioniert destilliert.
Ausbeute: 68,7g (81 % d. Th.) Sdp.: 95°-98°C/2 mbar
GC-Auswertung (20m DB-WAX, Innendurchmesser 0,18µm / 60-9-220°C Kaltaufgabesystem)
MS: m/z (%) = 212 (3), 183 (4), 169(29), 156 (4), 141 (5), 97 (15), 83 (8), 69 (6), 55 (48), 41 (100).

### Beispiel 2: Parfümkomposition (Riechstoffkomposition)

| | |
|---|---|
| Agrumex LC | 10,00 |
| Amarocit® 10%ig in DPG | 10,00 |
| Ambroxid kristallin | 10,00 |
| Basilikumöl | 10,00 |
| Calone 1951 10%ig in DPG | 10,00 |
| Cedernholzöl | 10,00 |
| Cedrol kristallin | 50,00 |
| Citral 10%ig in DPG | 10,00 |
| Citonellol | 5,00 |
| Cumarin | 10,00 |
| Dihydromyrcenol | 80,00 |
| Farenal® 10%ig in DPG | 5,00 |
| Geraniol | 80,00 |
| Geranylnitril | 40,00 |
| Hedion | 90,00 |
| Helional | 20,00 |
| Heliotropin | 5,00 |
| Hexenol cis-3 10%ig in DPG | 15,00 |
| Hexenylsalicylat cis-3 | 10,00 |
| Beta-Ionon | 5,00 |
| Iso E Super® | 180,00 |
| Isodamascon® 10%ig in DPG | 10,00 |
| Isoraldein 70 | 20,00 |
| Ketamber 10%ig in TEC | 25,00 |
| Lavandinöl Grosso, natürlich | 15,00 |
| Lilial | 20,00 |
| Linalool | 20,00 |
| Linalylacetat | 40,00 |
| Mandarinenöl brasilianisch grün | 50,00 |
| Timberol® | 40,00 |
| Vanillin | 5,00 |
| Veloutone 10%ig in DPG | 20,00 |
| Ysamber K® | 10,00 |
| TEC | 60,00 |
| | |
| Gesamt | 1000,00 |

Geruchsbeschreibung dieser nicht erfindungsgemäßen Parfümkomposition: frisch, holzig.

Nach Meinung der Parfümeure wird diese Parfümkomposition durch den Zusatz von 6 Gew.-% des Ketals aus Beispiel 1 frischer, strahlender, abgerundeter und harmonischer, wobei eine galbanumartige Note hinzukommt und die holzigen und blumigen Aspekte verstärkt werden. Das Ketal aus Beispiel 1 verleiht der Komposition einen eigenen Charakter und verbindet die unterschiedlichen geruchlichen Elemente.

### Beispiel 3: Parfümkomposition (Riechstoffkomposition)

| | |
|---|---|
| DPG | 167,00 |
| Amylsalicylat | 2,00 |
| Benzylacetat | 64,00 |
| Citronellol | 122,00 |
| Citral 10% ig in DPG | 2,00 |
| Cyclamenaldehyd | 10,00 |
| Dihydromyrcenol | 3,00 |
| Dimethylbenzylcarbinylacetat | 3,00 |
| Ethylsalicylat 10% ig in DPG | 2,00 |
| Eugenol | 3,00 |
| Indoflor 10%ig in DPG | 16,00 |
| Trifernal | 2,00 |
| Geraniol | 35,00 |
| Dihyd romethyljasmonat | 6,00 |
| Heliotropin | 4,00 |
| Hexylzimtaldehyd | 121,00 |
| Vertocitral | 4,00 |
| Hydroxycitronellal | 42,00 |
| Indol | 2,00 |
| Isobutylsalicylat | 6,00 |
| Lavandinöl Grosso, natürlich | 6,00 |
| Lactoscatone | 10,00 |
| Lilial | 190,00 |
| Linalool | 35,00 |
| Linalylacetat | 10,00 |
| Methylanthranilat 10%ig in DPG | 5,00 |
| Nerol | 10,00 |
| Orangenöl | 6,00 |
| Paraxonal | 4,00 |
| Phenylacetaldehyddimethylacetal | 6,00 |
| Phenylethylalkohol | 75,00 |
| Rosatol 10%ig in DPG | 6,00 |
| Sandelholzöl | 3,00 |
| Sandranol | 16,00 |
| TCD-Alcohol M | 2,00 |
| | |
| Gesamt | 1000,00 |

Geruchsbeschreibung dieser nicht erfindungsgemäßen Parfümkomposition: blumig, Maiglöckchen, mit fruchtigen Aspekten.

Nach Meinung der Parfümeure erwacht diese Parfümkomposition durch den Zusatz von 17 Gew.-% des Ketals aus Beispiel 1 zu neuem Leben. Der Eindruck von Fruchtigkeit wird erheblich verstärkt. Die Komposition wirkt strahlender, abgerundeter und harmonischer, wobei eine natürliche Note hinzukommt. Das Ketal aus Beispiel 1 verleiht der Komposition durch ihren Eigengeruch sowie durch ihre modifizierende und verstärkende Wirkung (Booster-Wirkung) einen eigenen Charakter und verbindet die unterschiedlichen geruchlichen Elemente.

### Beispiel 4: Shampoo

Das Ketal aus Beispiel 1 wurde in einer Dosierung von 0,5 Gew.-% in eine Shampoo-Grundmasse folgender Zusammensetzung eingearbeitet:

| | |
|---|---|
| Natriumlaurylethersulfat | 12% |
| (z.B. Texapon NSO, Fa. Cognis Deutschland GmbH) Cocamidopropylbetain | 2% |
| (z.B. Dehyton K, Fa. Cognis Deutschland GmbH) Natriumchlorid | 1,4% |
| Citronensäure | 1,3% |
| Phenoxyethanol, Methyl-, Ethyl-, Butyl-, und Propylparaben | 0,5% |
| Wasser | 82,8% |

Der pH-Wert der Shampoo-Grundmasse lag bei etwa 6. Hieraus wurden 100 mL einer 20 Gew.%-igen wässrigen Shampoo-Lösung hergestellt. In dieser Shampoo-Lösung wurden 2 Haarsträhnchen gemeinsam für 2 Minuten gewaschen und anschließend 20 Sekunden unter fließendem handwarmem Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt.

Geruchsbeschreibung beider Haarsträhnen: stark strahlend, galbanumartig, fruchtig.

### Beispiel 5: Weichspüler

Die Parfümkomposition aus Beispiel 2 (nach Zusatz von 6 Gew.-% des Ketals aus Beispiel 1) wurde in einer Dosierung von 0,5 Gew.-% in eine Weichspüler-Grundmasse folgender Zusammensetzung eingearbeitet:

| | |
|---|---|
| Quarternäres Ammoniummethosulfat (Esterquat), ca. 90% | 5,5% |
| (z.B. Rewoquat WE 18, Fa. Witco Surfactants GmbH) | |
| Alkyldimethylbenzylammoniumchlorid, ca. 50% | 0,2% |
| (z.B. Preventol R50, Fa. Bayer AG) | |
| Farblösung, ca. 1%-ig | 0,3% |
| Wasser | 94,0% |

Der pH-Wert der Weichspüler-Grundmasse lag im Bereich von 2 bis 3. Zwei Stofflappen wurden mit 370 g einer 1%-igen wässrigen Weichspüler-Lösung auf Basis der 0,5% Gew.-% der Parfümkomposition aus Beispiel 2 umfassenden Weichspüler-Grundmasse in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweisst, und einer zum Trocknen aufgehängt. Anschließend wurden beide Lappen durch ein Panel geruchlich beurteilt.

Geruchsbeschreibung beider Stofflappen: blumig, frisch, strahlend und holzige Aspekte mit leichten fruchtigen süßen Untertönen; abgerundeter und harmonischer Geruchseindruck.

### Beispiel 6: Waschpulver

Die Parfümölkomposition aus Beispiel 3 (nach Zusatz von 17 Gew.-% des Ketals aus Beispiel 1) wurde in einer Dosierung von 0,4 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer DOW CORNING 2-4248S | |
| POWDERED ANTIFOAM, Silikonöl auf Zeolith | |
| als Trägermaterial | 3,9 % |
| Zeolith 4A | 28,3 % |
| Na-Carbonat | 11,6 % |
| Na-Salz eines Copolymers aus Acryl- und Maleinsäure (Sokalan CP5) | 2,4 % |
| Na-Silikat | 3,0 % |
| Carboxymethylcellulose | 1,2 % |
| Dequest 2066 | 2,8 % |
| ([[(Phosphonomethyl)imino]bis[(ethylennitrilo)bis (methylen)]]tetrakis-phosphonsäure, Natriumsalz) | |
| Optischer Aufheller | 0,2 % |
| Na-Sulfat | 6,5 % |
| Protease | 0,4 % |
| Natriumperborat-tetrahydrat | 22,0 % |
| TAED | 1,0% |

Zwei Stofflappen wurden mit 370 g einer 1%-igen wässrigen Waschpulverlauge auf Basis des 0,4 Gew.-% der Parfümölkomposition aus Beispiel 3 umfassenden Waschpulver-Grundmasse (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweisst, und einer zum Trocknen aufgehängt. Anschließend wurden beide Lappen durch ein Panel geruchlich beurteilt.

Geruchsbeschreibung jeweils: stark, strahlend, galbanumartig mit natürlicher Note und fruchtigen Untertönen; abgerundeter und harmonischer Geruchseindruck.

## Patentansprüche

1. Verwendung einer Verbindung der Formel (I), 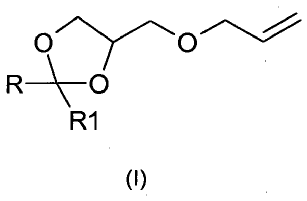 wobei R und R1
- jeweils unabhängig voneinander Wasserstoff oder einen organischen Rest mit 1 bis 15 Kohlenstoffatomen bedeuten
oder
- zusammen mit dem Kohlenstoffatom, an das sie gebunden sind, einen Ring bilden,
als Riech- oder Aromastoff.

2. Verwendung einer Verbindung der Formel (I) gemäß Anspruch 1 als Riech- oder Aromastoff
(i) mit Galbanum-Note
und/oder
(ii) zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, süß, erdig, fettig, metallisch und balsamisch, vorzugsweise zumindest einer der Noten grün und/oder fruchtig.

3. Verwendung nach einem der vorangehenden Ansprüche, wobei R und R1
- jeweils unabhängig voneinander Wasserstoff oder einen organischen Rest mit 1 bis 15 Kohlenstoffatomen bedeuten, wobei
(i) unabhängig voneinander einer der Reste R und R1 Wasserstoff und der andere Rest einen organischen Rest mit 1 bis 15 Kohlenstoffatomen bedeutet
oder
(ii) unabhängig voneinander sowohl R als auch R1 einen organischen Rest mit 1 bis 15 Kohlenstoffatomen bedeuten,
oder
- zusammen mit dem Kohlenstoffatom, an das sie gebunden sind, einen Ring mit 5, 6, 7 oder 8 Ring-Kohlenstoffatomen bilden.

4. Verwendung nach einem der vorangehenden Ansprüche, wobei R und R1
jeweils unabhängig voneinander Wasserstoff oder einen organischen Rest mit 1 bis 15 Kohlenstoffatomen bedeuten, wobei der, einer oder jeder organische Rest ein
gesättigter oder ungesättigter Rest ist,
und/oder
ein aromatischer oder aliphatischer Rest ist,
und/oder
ein alicyclischer oder nicht-cyclischer Rest ist,
und/oder
ein verzweigter oder unverzweigter Rest ist,
und/oder
der, einer oder jeder organische Rest 0, 1, 2 oder 3 Sauerstoff-Atome enthält.

5. Verwendung nach einem der vorangehenden Ansprüche, wobei R oder R1 oder beide unabhängig voneinander einen organischen, aliphatischen Rest mit 1 bis 15 Kohlenstoffatomen bedeuten.

6. Verwendung nach Anspruch 5, wobei der jeweilige aliphatische Rest ausgewählt ist aus der Gruppe bestehend aus:
Methyl, Ethyl, n-Propyl, iso-Propyl, n-Butyl, sec-Butyl, iso-Butyl, tert-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl,
But-3-enyl, 2,6-Dimethylhept-5-enyl, 3,5-Dimethylhex-4-enyl, 4-Mehtylpent-3-enyl, 2,6-Dimethylhepta-1,5-dienyl, 2-Methylpropenyl.

7. Verwendung nach einem der vorangehenden Ansprüche, wobei die Verbindung ausgewählt ist aus der Gruppe bestehend aus den Verbindungen der Formeln 3, 14, 15, 16, 17, 18, 19, 20, 21, 22 und 23:

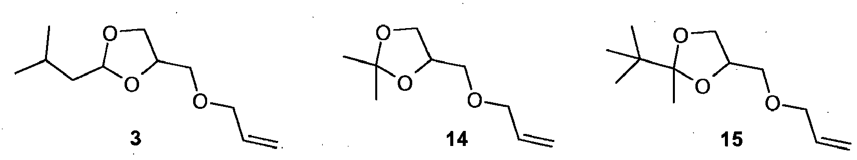

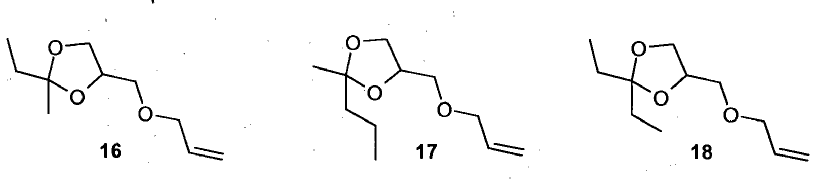

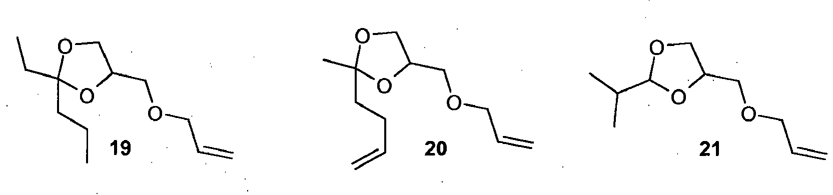

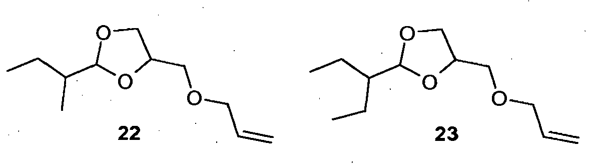

8. Verwendung nach einem der Ansprüche 1 bis 3, wobei R und R1 zusammen mit dem Kohlenstoffatom, an das sie gebunden sind, einen Ring bilden, der
eine oder zwei Doppelbindungen umfasst
und/oder
1, 2 oder 3 Sauerstoffatome enthält
und/oder
im Bereich der Reste R und R1 unsubstituiert oder substituiert ist mit einer oder mehreren verzweigten oder unverzweigten Alkylgruppen, Alkenylgruppen, Cycloalkylgruppen, Cycloalkenylgruppen, Arylgruppen, Arylalkylgruppen, Alkoxyalkylgruppen oder Alkoxyarylgruppen
und/oder
zusammen mit seinen gegebenenfalls vorhandenen Substituenten 5 bis 30 Kohlenstoff-Atome, bevorzugt 5 bis 15 Kohlenstoff-Atome, umfasst.

9. Verwendung nach Anspruch 8, wobei der Ring ein Carbocyclus oder ein Heterocyclus ist.

10. Verwendung nach Anspruch 9, wobei der Ring ein aliphatischer Carbocyclus oder Heterocyclus ist.

11. Verwendung nach einem der Ansprüche 8 bis 10, wobei R und R1
zusammen mit dem Kohlenstoffatom, an das sie gebunden sind, einen Ring bilden, der im Bereich der Reste R und R1 substituiert ist mit einer oder mehreren Substituenten ausgewählt aus der Gruppe bestehend aus: Methyl, Ethyl, n-Propyl, iso-Propyl, n-Butyl, sec-Butyl, iso-Butyl, tert-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, But-3-enyl, 2,6-Dimethylhept-5-enyl, 3,5-Dimethylhex-4-enyl, 4-Mehtylpent-3-enyl, 2,6-Dimethylhepta-1,5-dienyl, 2-Methylpropenyl.

12. Verwendung nach einem der Ansprüche 8 bis 11, wobei die Verbindung ausgewählt ist aus der Gruppe bestehend aus den Verbindungen mit den Formeln 2, 4, 8, 1, 9, 10, 11, 7, 5, 6, 12 und 13:

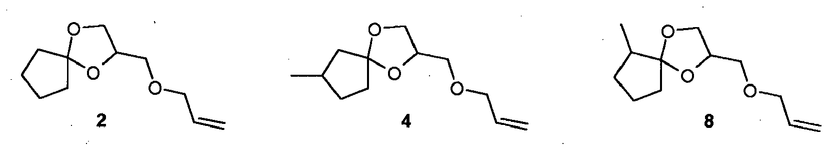

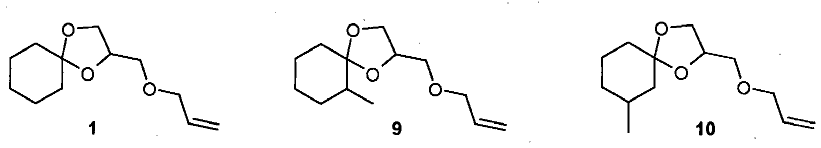

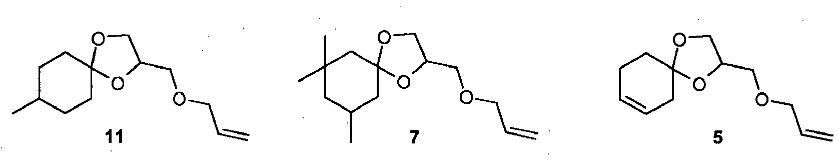

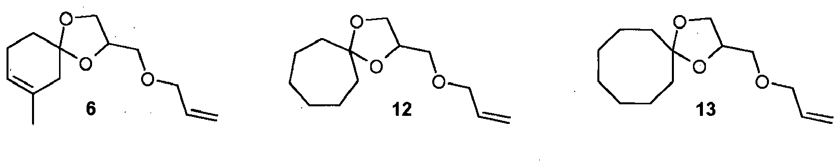

13. Verbindung der Formel (I) 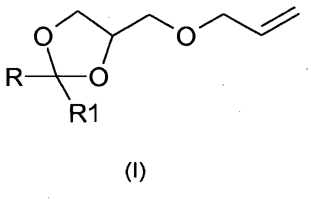 wobei
- unabhängig voneinander einer der Reste R und R1 Wasserstoff und der andere Rest Prop-2-en-1-yl, n-Propyl oder iso-Propyl oder einen aliphatischen, nicht-cyclischen Rest mit 4 bis 15 Kohlenstoffatomen bedeutet, der gesättigt oder ungesättigt ist und gleichzeitig verzweigt oder unverzweigt ist,
oder
- unabhängig voneinander einer der Reste R und R1 Methyl und der andere Rest einen aliphatischen, nicht-cyclischen, ungesättigten Rest mit 2 bis 15 Kohlenstoffatomen bedeutet, der verzweigt oder unverzweigt ist,
oder
- unabhängig voneinander sowohl R als auch R1 einen aliphatischen, nicht-cyclischen Rest mit 2 bis 15 Kohlenstoffatomen bedeuten, der jeweils gesättigt oder ungesättigt ist und gleichzeitig jeweils verzweigt oder unverzweigt ist,
oder
- zusammen mit dem Kohlenstoffatom, an das sie gebunden sind, einen Ring mit 5, 7 oder 8 Ring-Kohlenstoffatomen bilden,
oder
- zusammen mit dem Kohlenstoffatom, an das sie gebunden sind, einen Ring mit 6 Ring-Kohlenstoffatomen bilden, wobei der Ring im Bereich der Reste R und R1 substituiert und/oder ungesättigt ist,
oder
- zusammen mit dem Kohlenstoffatom, an das sie gebunden sind, entweder
(i) einen Ring mit genau einem Ring-Sauerstoffatom bilden, wobei der Ring (a) 6 Ring-Kohlenstoffatome besitzt und das Sauerstoffatom nicht an das Kohlenstoffatom gebunden ist, an das R und R1 gebunden sind oder (b) 5, 7 oder 8 Ring-Kohlenstoffatome besitzt oder
(ii) einen Ring mit mehreren Ring-Sauerstoffatomen bilden.

14. Verbindung nach Anspruch 13, wobei R und R1 zusammen mit dem Kohlenstoffatom, an das sie gebunden sind, einen Ring bilden, der
insgesamt 5 bis 16 Kohlenstoff-Atome umfasst
und/oder
eine oder zwei Doppelbindungen umfasst
und/oder
1, 2 oder 3 Sauerstoffatome enthält
und/oder
im Bereich der Reste R und R1 unsubstituiert oder substituiert ist mit einer oder mehreren verzweigten oder unverzweigten Alkylgruppen, Alkenylgruppen, Cycloalkylgruppen, Cycloalkenylgruppen, Arylgruppen, Arylalkylgruppen, Alkoxyalkylgruppen oder Alkoxyarylgruppen.

15. Verbindung nach Anspruch 14, wobei der Ring ein Carbocyclus oder ein Heterocyclus ist.

16. Verbindung nach Anspruch 15, wobei der Ring ein aliphatischer Carbocyclus oder Heterocyclus ist.

17. Verbindung nach einem der Ansprüche 13 bis 16, wobei R und R1 zusammen mit dem Kohlenstoffatom, an das sie gebunden sind, einen Ring bilden, der im Bereich der Reste R und R1 substituiert ist mit einer oder mehreren Substituenten ausgewählt aus der Gruppe bestehend aus: Methyl, Ethyl, n-Propyl, iso-Propyl, n-Butyl, sec-Butyl, iso-Butyl, tert-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, But-3-enyl, 2,6-Dimethylhept-5-enyl, 3,5-Dimethylhex-4-enyl, 4-Mehtylpent-3-enyl, 2,6-Dimethylhepta-1,5-dienyl, 2-Methylpropenyl.

18. Verbindung nach einem der Ansprüche 13 bis 17, wobei die Verbindung ausgewählt ist aus der Gruppe bestehend aus den Verbindungen mit den Formeln 2, 3, 4, 5, 6, 7, 8, 9, 10, 11, 12, 13, 18, 19, 20, 21, 22 und 23:

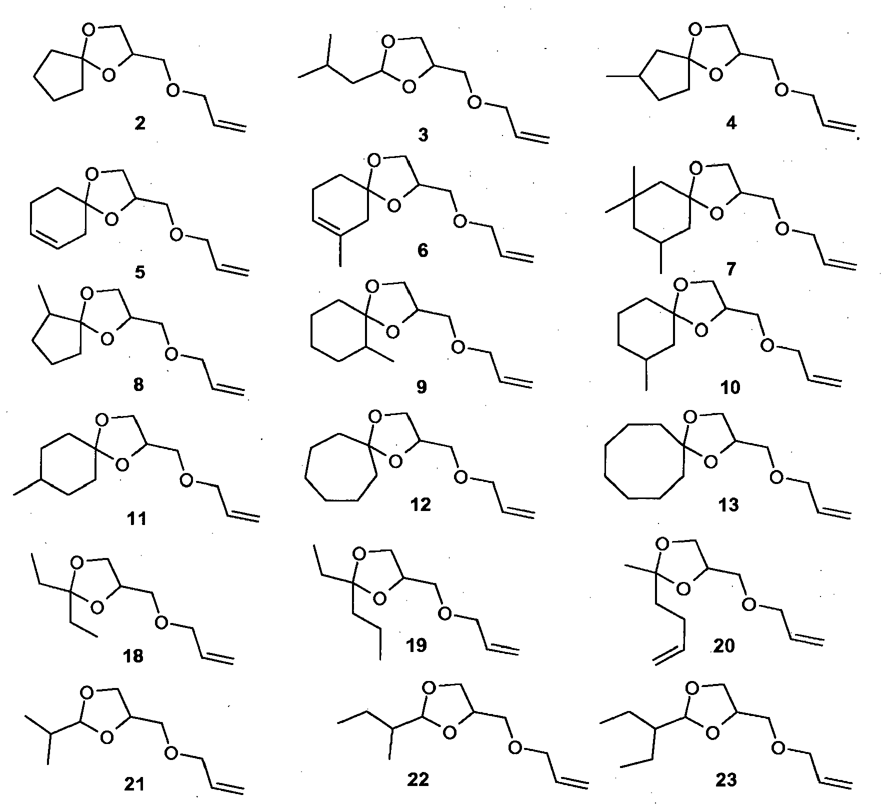

19. Riech- oder Aromastoffkomposition umfassend:
a) eine oder mehrere Verbindungen wie in einem der vorangehenden Ansprüche 1 bis 12 oder 13 bis 18 definiert sowie
b) einen oder mehrere weitere Riech- oder Aromastoffe,
(i) wobei der oder die weiteren Riech- oder Aromastoffe einen holzigen, fruchtigen und/oder blumigen Geruch und/oder Geschmack vermitteln
oder
(ii) wobei der oder die weiteren Riech- oder Aromastoffe einen anderen Geruch und/oder Geschmack vermitteln.

20. Riech- oder Aromastoffkomposition nach Anspruch 19, wobei das Gewichtsverhältnis der Gesamtmenge an Verbindungen wie in einem der vorangehenden Ansprüche 1 bis 12 oder 13 bis 18 definiert zu der Gesamtmenge an weiteren Riech- oder Aromastoffen im Bereich von 1 : 1000 bis 1 : 0,5 liegt.

21. Riech- oder Aromastoffkomposition nach Anspruch 19 oder 20, wobei die Gesamtmenge an Verbindungen wie in einem der vorangehenden Ansprüche 1 bis 12 oder 13 bis 18 definiert im Bereich von 0,0001 bis 99,9 Gew.-%, vorzugsweise 0,001 bis 99,5 Gew.-%, besonders bevorzugt 0,01 bis 99 Gew.-% liegt, jeweils bezogen auf die Gesamtmenge der Riech- oder Aromastoffkomposition.

22. Riech- oder Aromastoffkomposition nach einem der Ansprüche 19 bis 21, wobei die eingesetzte Gesamtmenge der oder den Verbindungen wie in einem der vorangehenden Ansprüche 1 bis 12 oder 13 bis 18 definiert ausreicht, um den Geruch und/oder Geschmack der weiteren Riech- oder Aromastoffe in Richtung grün, krautig, frisch, fruchtig, blumig, holzig, süß, erdig, fettig, metallisch und balsamisch, vorzugsweise in Richtung grün und/oder fruchtig, bevorzugt in Richtung Galbanum, zu modifizieren.

23. Verwendung einer Riech- oder Aromastoffkomposition nach einem der Ansprüche 19 bis 22
(A) als Riech- oder Aromastoffkomposition
(a)
(i) mit Galbanum-Note
und/oder
(ii) zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, süß, erdig, fettig, metallisch und balsamisch, vorzugsweise zumindest einer der Noten grün und/oder fruchtig,
und/oder
(b) (i) mit fruchtiger und/oder mit blumiger Note,
und/oder
(ii) zum Vermitteln, Modifizieren und/oder Verstärken einer oder mehrerer Geruchs- und/oder Geschmacksnoten ausgewählt aus der Gruppe bestehend aus den Noten holzig, fruchtig und/oder blumig,
und/oder
(c) mit blumiger, fruchtiger, galbanumartiger Note.
und/oder
(B) als Bestandteil eines parfümierten oder aromatisierten Artikels.

24. Parfümierter oder aromatisierter Artikel, umfassend
- eine oder mehrere Verbindungen der Formel (I) Verbindungen wie in einem der vorangehenden Ansprüche 13 bis 18 definiert oder einer Riech- oder Aromastoffkomposition nach einem der Ansprüche 19 bis 22, vorzugsweise in einer sensorisch wirksamen Menge,
und
- einen oder mehrere weitere Zusatz-, Hilfs- und/oder Wirkstoffe, vorzugsweise zwei, drei, vier, fünf oder mehr Zusatz-, Hilfs- und/oder Wirkstoffe.

25. Parfümierter oder aromatisierter Artikel nach Anspruch 24, wobei der parfümierte Artikel ausgewählt ist aus der Gruppe bestehend aus:
Parfüm-Extraits, Eau de Parfums, Eau de Toilettes, Rasierwässer, Eau de Colognes, Preshave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -Iotionen, Fußcremes und -lotionen, Enthaarungscremes und -lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

26. Parfümierter oder aromatisierter Artikel nach Anspruch 24 oder 25, wobei die Gesamtmenge an Verbindungen wie in einem der vorangehenden Ansprüche 1 bis 12 oder 13 bis 18 definiert im Bereich von 0,00001 bis 10 Gew.-% liegt, vorzugsweise im Bereich von 0,0001 bis 5 Gew.-%, besonders bevorzugt im Bereich von 0,001 bis 2 Gew.-%, am meisten bevorzugt 0,005 bis 1 Gew.-%, jeweils bezogen auf die Gesamtmasse des Artikels.

27. Verfahren zum Vermitteln, Modifizieren und/oder Verstärken einer oder mehrerer Geruchs- und/oder Geschmacksnoten ausgewählt aus der Gruppe bestehend aus den Noten grün, krautig, frisch, fruchtig, blumig, holzig, süß, erdig, fettig, metallisch und balsamisch, vorzugsweise zumindest einer der Noten grün und/oder fruchtig, bevorzugt einer Galbanum-Note, mit folgenden Schritten
- Bereitstellen einer oder mehrerer Verbindungen wie in einem der vorangehenden Ansprüche 1 bis 12 oder 13 bis 18 definiert oder einer Riech- oder Aromastoffkomposition nach einem der Ansprüche 19 bis 22,
- Bereitstellen eines Artikels,
- In Kontakt bringen des Artikels mit einer sensorisch wirksamen Menge von der oder den Verbindungen wie in einem der vorangehenden Ansprüche 1 bis 12 oder 13 bis 18 definiert bzw. von der Riech- oder Aromastoffkomposition nach einem der Ansprüche 19 bis 22.

28. Verfahren nach Anspruch 27, wobei nach dem in Kontakt bringen (i) ein parfümierter Artikel resultiert oder (ii) aus dem dann vorliegenden Erzeugnis hergestellt wird, der ausgewählt ist aus der Gruppe bestehend aus:
Parfüm-Extraits, Eau de Parfums, Eau de Toilettes, Rasierwässer, Eau de Colognes, Preshave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

## Claims

1. Use of a compound of formula (I), 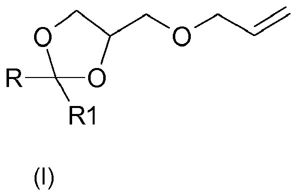 wherein R and R1
- in each case independent of each other denote hydrogen or an organic group having 1 to 15 carbon atoms
or
- together with the carbon atom to which they are bound, form a ring,
as fragrance or flavor.

2. Use of a compound of formula (I) according to claim 1 as fragrance or flavor
(i) with a Galbanum impression
and/or
(ii) for conveying, modifying and/or enhancing one or more fragrance and/or flavor impression(s) selected from the group consisting of the impressions green, herbaceous, fresh, fruity, flowery, woody, sweet, earthy, fatty, metallic and balsamic, preferably at least one of the impressions green and/or fruity.

3. Use according to one of the preceding claims, wherein R and R1
- in each case independent of each other denote hydrogen or an organic group having 1 to 15 carbon atoms, wherein
(i) independent of each other one of the groups R and R1 denotes hydrogen and the other group denotes an organic group having 1 to 15 carbon atoms
or
(ii) independent of each other, both, R and R1 denote an organic group having 1 to 15 carbon atoms,
or
- together with the carbon atom to which they are bound, form a ring having 5, 6, 7 or 8 ring carbon atoms.

4. Use according to one of the preceding claims, wherein R and R1 in each case independent of each other denote hydrogen or an organic group having 1 to 15 carbon atoms, wherein the, one or each organic group is a
saturated or unsaturated group,
and/or
an aromatic or aliphatic group,
and/or
an alicyclic or non-cyclic group,
and/or
a branched or unbranched group,
and/or
the, one or each organic group comprises 0, 1, 2 or 3 oxygen atoms.

5. Use according to one of the preceding claims, wherein R or R1 or both independent of each other denote an organic, aliphatic group having 1 to 15 carbon atoms.

6. Use according to claim 5, wherein the respective aliphatic group is selected from the group consisting of:
methyl, ethyl, n-propyl, iso-propyl, n-butyl, sec-butyl, iso-butyl, tert-butyl, n-pentyl, 2-pentyl, 3-pentyl, 2-methyl butyl, 3-methyl butyl, 3-methyl but-2-yl, 2-methyl but-2-yl, 2,2-dimethyl propyl, n-hexyl, 1-methyl pentyl, 2-methyl pentyl, 3-methyl pentyl, 4-methyl pentyl, 1,1-dimethyl butyl, 1,2-dimethyl butyl, 1,3-dimethyl butyl, 2,2-dimethyl butyl, 2,3-dimethyl butyl, 3,3-dimethyl butyl, 1-ethyl butyl, 2-ethyl butyl, 1,1,2-trimethyl propyl, 1,2,2-trimethyl propyl, 1-ethyl-1-methyl propyl and 1-ethyl-2-methyl propyl, but-3-enyl, 2,6-dimethyl hept-5-enyl, 3,5-dimethyl hex-4-enyl, 4-mehtyl pent-3-enyl, 2,6-dimethyl hepta-1,5-dienyl, 2-methyl propenyl.

7. Use according to one of the preceding claims, wherein the compound is selected from the group consisting of the compounds of formulas 3, 14, 15, 16, 17, 18, 19, 20, 21, 22 and 23:

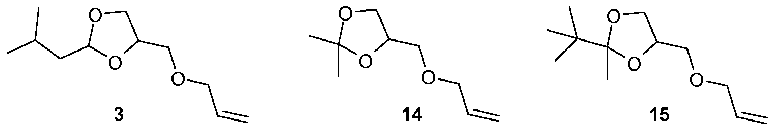

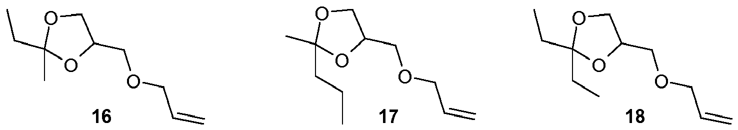

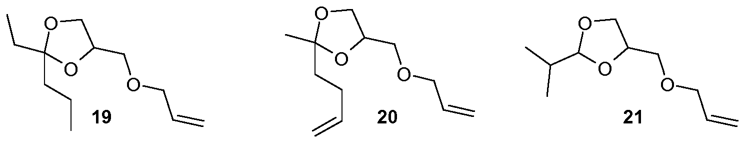

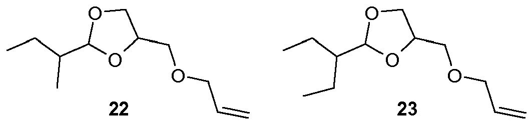

8. Use according to one of claims 1 to 3, wherein R and R1 together with the carbon atom to which they are bound, form a ring, which
comprises one or two double bonds
and/or
contains 1, 2 or 3 oxygen atoms
and/or
is, in the region of the groups R and R1, unsubstituted or substituted with one or more branched or unbranched alkyl groups, alkenyl groups, cycloalkyl groups, cycloalkenyl groups, aryl groups, arylalkyl groups, alkoxyalkyl groups or alkoxyaryl groups
and/or together with its optionally present substituents, comprises 5 to 30 carbon atoms, preferably 5 to 15 carbon atoms.

9. Use according to claim 8, wherein the ring is a carbocycle or a heterocycle.

10. Use according to claim 9, wherein the ring is an aliphatic carbocycle or heterocycle.

11. Use according to one of claims 8 to 10, wherein R and R1
together with the carbon atom to which they are bound, form a ring, which is substituted in the region of the groups R and R1 with one or more substituents selected from the group consisting of: methyl, ethyl, n-propyl, iso-propyl, n-butyl, sec-butyl, iso-butyl, tert-butyl, n-pentyl, 2-pentyl, 3-pentyl, 2-methyl butyl, 3-methyl butyl, 3-methyl but-2-yl, 2-methyl but-2-yl, 2,2-dimethyl propyl, n-hexyl, 1-methyl pentyl, 2-methyl pentyl, 3-methyl pentyl, 4-methyl pentyl, 1,1-dimethyl butyl, 1,2-dimethyl butyl, 1,3-dimethyl butyl, 2,2-dimethyl butyl, 2,3-dimethyl butyl, 3,3-dimethyl butyl, 1-ethyl butyl, 2-ethyl butyl, 1,1,2-trimethyl propyl, 1,2,2-trimethyl propyl, 1-ethyl-1-methyl propyl and 1-ethyl-2-methyl propyl, but-3-enyl, 2,6-dimethyl hept-5-enyl, 3,5-dimethyl hex-4-enyl, 4-mehtyl pent-3-enyl, 2,6-dimethyl hepta-1,5-dienyl, 2-methyl propenyl.

12. Use according to one of the claims 8 to 11, wherein the compound is selected from the group consisting of the compounds of the formulas 2, 4, 8, 1, 9, 10, 11, 7, 5, 6, 12 and 13:

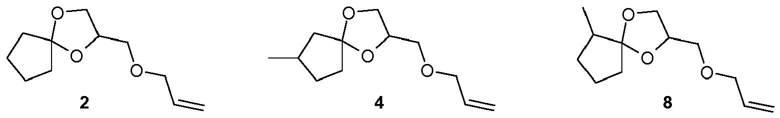

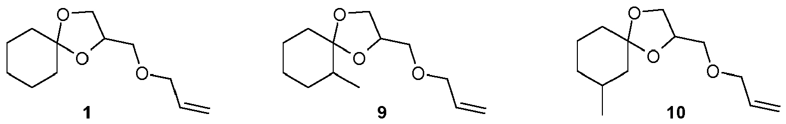

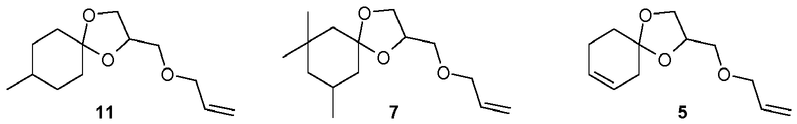

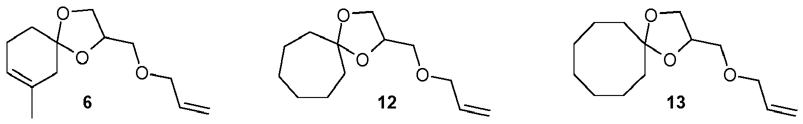

13. Compound of formula (I) 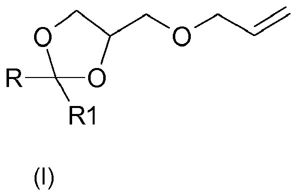 wherein
- independent of each other one of the groups R and R1 denotes hydrogen and the other group denotes prop-2-en-1-yl, n-propyl or iso-propyl or an aliphatic, non-cyclic group having 4 to 15 carbon atoms, which is saturated or unsaturated and at the same time branched or unbranched,
or
- independent of each other one of the groups R and R1 denotes methyl and the other group denotes an aliphatic, non-cyclic, unsaturated group having 2 to 15 carbon atoms, which is branched or unbranched,
or
- independent of each other both, R and R1 denote an aliphatic, non-cyclic group having 2 to 15 carbon atoms, which is saturated or unsaturated, respectively, and at the same time branched or unbranched, respectively,
or
- together with the carbon atom to which they are bound, form a ring with 5, 7 or 8
ring carbon atoms,
or
- together with the carbon atom to which they are bound, form a ring with 6 ring carbon atoms, wherein the ring is substituted and/or unsaturated in the region of the groups R and R1,
or
- together with the carbon atom to which they are bound, either
(i) form a ring with exactly one ring oxygen atom, wherein the ring (a) comprises 6 ring carbon atoms and the oxygen atom is not bound to the carbon atom to which R and R1 are bound or (b) comprises 5, 7 or 8 ring carbon atoms or
(ii) form a ring with several ring oxygen atoms.

14. Compound according to claim 13, wherein R and R1 together with the carbon atom to which they are bound, form a ring, which
in total comprises 5 to 16 carbon atoms
and/or
comprises one or two double bonds
and/or
contains 1, 2 or 3 oxygen atoms
and/or
is, in the region of the groups R and R1, unsubstituted or substituted with one or more branched or unbranched alkyl groups, alkenyl groups, cycloalkyl groups, cycloalkenyl groups, aryl groups, arylalkyl groups, alkoxyalkyl groups or alkoxyaryl groups.

15. Compound according to claim 14, wherein the ring is a carbocycle or a heterocycle.

16. Compound according to claim 15, wherein the ring is an aliphatic carbocycle or heterocycle.

17. Compound according to one of the claims 13 to 16, wherein R and R1 together with the carbon atom to which they are bound, form a ring, which is substituted in the region of the groups R and R1 with one or more substituents selected from the group consisting of: methyl, ethyl, n-propyl, iso-propyl, n-butyl, sec-butyl, iso-butyl, tert-butyl, n-pentyl, 2-pentyl, 3-pentyl, 2-methyl butyl, 3-methyl butyl, 3-methyl but-2-yl, 2-methyl but-2-yl, 2,2-dimethyl propyl, n-hexyl, 1-methyl pentyl, 2-methyl pentyl, 3-methyl pentyl, 4-methyl pentyl, 1,1-dimethyl butyl, 1,2-dimethyl butyl, 1,3-dimethyl butyl, 2,2-dimethyl butyl, 2,3-dimethyl butyl, 3,3-dimethyl butyl, 1-ethyl butyl, 2-ethyl butyl, 1,1,2-trimethyl propyl, 1,2,2-trimethyl propyl, 1-ethyl-1-methyl propyl and 1-ethyl-2-methyl propyl, but-3-enyl, 2,6-dimethyl hept-5-enyl, 3,5-dimethyl hex-4-enyl, 4-mehtyl pent-3-enyl, 2,6-dimethyl hepta-1,5-dienyl, 2-methyl propenyl.

18. Compound according to one of the claims 13 to 17, wherein the compound is selected from the group consisting of the compounds of fomulas 2, 3, 4, 5, 6, 7, 8, 9, 10, 11, 12, 13, 18, 19, 20, 21, 22 and 23:

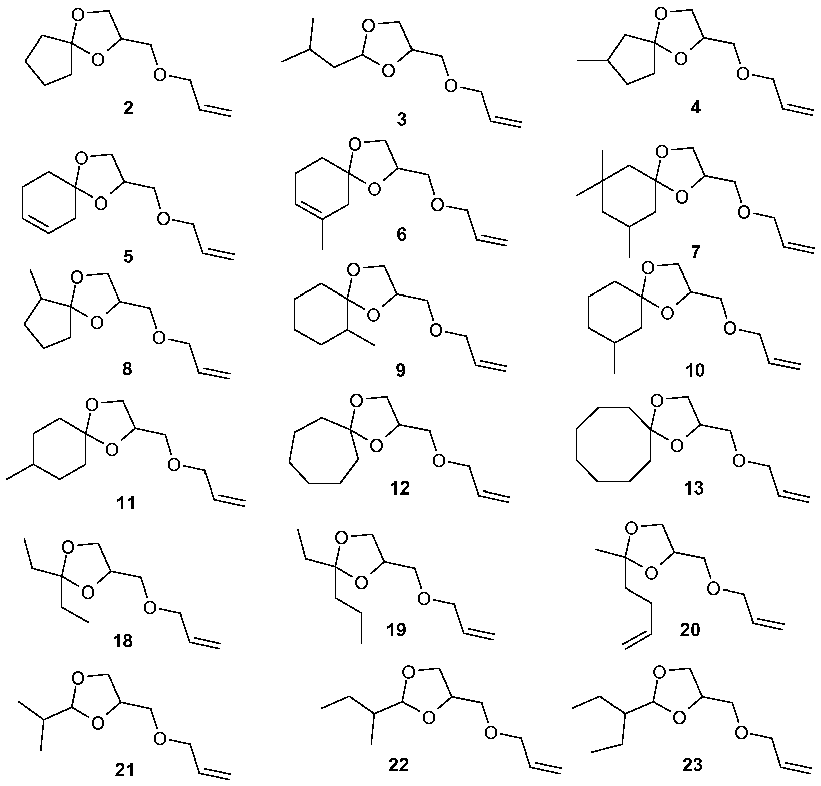

19. Fragrance or flavor composition, comprising:
a) one or more compounds as defined in one of the preceding claims 1 to 12 or 13 to 18, as well as
b) one or more further fragrance(s) or flavor(s),
(i) wherein the further fragrance(s) or flavor(s) convey a woody, fruity and/or flowery smell and/or taste
or
(ii) wherein the further fragrance(s) or flavor(s) convey a different smell and/or taste.

20. Fragrance or flavor composition according to claim 19, wherein the weight ratio of the total amount of compounds as defined in one of the preceding claims 1 to 12 or 13 to 18 to the total amount of further fragrance(s) or flavor(s) is in the range of 1 : 1000 to 1 : 0.5.

21. Fragrance or flavor composition according to claim 19 or 20, wherein the total amount of compounds as defined in one of the preceding claims 1 to 12 or 13 to 18 is in the range from 0.0001 to 99.9 wt.-%, preferably 0.001 to 99.5 wt.-%, particularly preferably 0.01 to 99 wt.-%, in each case with respect to the total amount of the fragrance or flavor composition.

22. Fragrance or flavor composition according to one of the claims 19 to 21, wherein the total amount of the compound(s) as defined in one of the preceding claims 1 to 12 or 13 to 18 used, is sufficient to modify the smell and/or the taste of the further fragrance(s) or flavor(s) towards green, herbaceous, fresh, fruity, flowery, woody, sweet, earthy, fatty, metallic and balsamic, preferably towards green and/or fruity, preferably towards Galbanum.

23. Use of a fragrance or flavor composition according to one of claims 19 to 22
(A) as fragrance or flavor composition
(a)
(i) with a Galbanum impression
and/or
(ii) for conveying, modifying and/or enhancing one or more fragrance and/or flavor impression(s) selected from the group consisting of the impressions green, herbaceous, fresh, fruity, flowery, woody, sweet, earthy, fatty, metallic and balsamic, preferably at least one of the impressions green and/or fruity,
and/or
(b) (i) with a fruity and/or flowery impression,
and/or
(ii) for conveying, modifying and/or enhancing one or more fragrance and/or flavor impression(s) selected from the group consisting of the impressions woody, fruity and/or flowery,
and/or
(c) with flowery, fruity, galbanum like impression,
and/or
(B) as a component of a perfumed or flavored article.

24. Perfumed or flavored article, comprising
- one or more compound(s) of formula (I), compounds as defined in one of the preceding claims 13 to 18 or a fragrance or flavor composition according to one of the claims 19 to 22, preferably in a sensorically effective amount,
and
- one or more further additives, excipients and/or active agents, preferably two, three, four, fife or more additives, excipients and/or active agents.

25. Perfumed or flavored article according to claim 24, wherein the perfumed or flavored article is selected from the group consisting of:
perfume extraits, eau de parfums, eau de toilettes, aftershave, eau de colognes, pre-shave products, splash colognes, perfumed refreshing wipes, acid, alkaline or neutral detergents, textile fresheners, ironing aids, liquid laundry soap, powder laundry soap, laundry pretreatment agents, softeners, washing soaps, washing tablets, disinfectants, surface disinfectants, air fresheners, aerosol sprays, waxes and polishes, body care products, hand crèmes and lotions, foot crèmes and lotions, hair removal crèmes and lotions, aftershave crèmes and lotions, tanning crèmes and lotions, hair care products, deodorants, antiperspirants, products of decorative cosmetics, candles, lamp oil, incense sticks, insecticides, repellents and propellants.

26. Perfumed or flavored article according to claim 24 or 25, wherein the total amount of compounds as defined in one of the preceding claims 1 to 12 or 13 to 18 is in the range from 0.00001 to 10 wt.-%, preferably in the range from 0.0001 to 5 wt.-%, particularly preferably in the range from 0.001 to 2 wt.-%, most preferably 0.005 to 1 wt.-%, in each case with respect to the total weight of the article.

27. Method for conveying, modifying and/or enhancing one or more fragrance and/or flavor impression(s) selected from the group consisting of the impressions green, herbaceous, fresh, fruity, flowery, woody, sweet, earthy, fatty, metallic and balsamic, preferably at least one of the impressions green and/or fruity, preferred a Galbanum impression, with the following steps
- providing one or more compound(s) as defined in one of the preceding claims 1 to 12 or 13 to 18 or a fragrance or flavor composition according to one of claims 19 to 22,
- providing an article,
- contacting the article with a sensorically effective amount of compound(s) as defined in one of the preceding claims 1 to 12 or 13 to 18 or a fragrance or flavor composition according to one of claims 19 to 22.

28. Method according to claim 27, wherein after the contacting (i) a perfumed article results or (ii) or is produced from the then present product, which is selected from the group consisting of:
perfume extraits, eau de parfums, eau de toilettes, aftershave, eau de colognes, pre-shave products, splash colognes, perfumed refreshing wipes, acid, alkaline or neutral detergents, textile fresheners, ironing aids, liquid laundry soap, powder laundry soap, laundry pretreatment agents, softeners, washing soaps, washing tablets, disinfectants, surface disinfectants, air fresheners, aerosol sprays, waxes and polishes, body care products, hand crèmes and lotions, foot crèmes and lotions, hair removal crèmes and lotions, aftershave crèmes and lotions, tanning crèmes and lotions, hair care products, deodorants, antiperspirants, products of decorative cosmetics, candles, lamp oil, incense sticks, insecticides, repellents and propellants.

## Revendications

1. Utilisation d'un composé de formule (I), 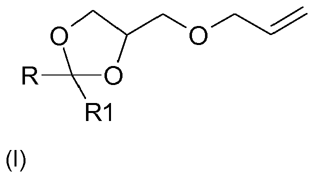 dans laquelle R et R1
- signifient chacun, indépendamment l'un de l'autre, l'hydrogène ou un radical organique ayant 1 à 15 atomes de carbone,
ou
- forment un cycle conjointement avec l'atome de carbone auquel ils sont liés, en tant que substance odoriférante ou aromatisante.

2. Utilisation d'un composé de formule (I) selon la revendication 1, en tant que substance odoriférante ou aromatisante
(i) ayant une note de galbanum
et/ou
(ii) pour conférer, modifier et/ou intensifier une ou plusieurs notes olfactives et/ou gustatives choisies dans le groupe constitué par les notes verte, herbacée, fraîche, fruitée, fleurie, ligneuse, douce, terreuse, graisseuse, métallique et balsamique, de préférence au moins l'une des notes verte et/ou fruitée.

3. Utilisation selon l'une quelconque des revendications précédentes, dans laquelle R et R1
- signifient chacun, indépendamment l'un de l'autre, l'hydrogène ou un radical organique ayant 1 à 15 atomes de carbone, où
(i) indépendamment l'un de l'autre, l'un des radicaux R et R1 signifie l'hydrogène et l'autre radical signifie un radical organique ayant 1 à 15 atomes de carbone
ou,
(ii) indépendamment l'un de l'autre, aussi bien R que R1 signifient un radical organique ayant 1 à 15 atomes de carbone,
ou
- forment, conjointement avec l'atome de carbone auquel ils sont liés, un cycle ayant 5, 6, 7 ou 8 atomes de carbone cycliques.

4. Utilisation selon l'une quelconque des revendications précédentes, dans laquelle R et R1
- signifient chacun, indépendamment l'un de l'autre, l'hydrogène ou un radical organique ayant 1 à 15 atomes de carbone, le, un ou chaque radical organique
étant un radical saturé ou insaturé
et/ou
étant un radical aromatique ou aliphatique
et/ou
étant un radical alicyclique ou non cyclique
et/ou
étant un radical ramifié ou non ramifié
et/ou
le, un ou chaque radical organique contenant 0, 1, 2 ou 3 atomes d'oxygène.

5. Utilisation selon l'une quelconque des revendications précédentes, dans laquelle R ou R1 ou les deux, indépendamment l'un de l'autre, signifient un radical organique aliphatique ayant 1 à 15 atomes de carbone.

6. Utilisation selon la revendication 5, dans laquelle le radical aliphatique respectif est choisi dans le groupe constitué par:
les groupes méthyle, éthyle, n-propyle, isopropyle, n-butyle, sec-butyle, isobutyle, tert-butyle, n-pentyle, 2-pentyle, 3-pentyle, 2-méthylbutyle, 3-méthylbutyle, 3-méthylbut-2-yle, 2-méthylbut-2-yle, 2,2-diméthylpropyle, n-hexyle, 1-méthylpentyl, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle et 1-éthyl-2-méthylpropyle, but-3-ényle, 2,6-diméthylhept-5-ényle, 3,5-diméthylhex-4-ényle, 4-méthtylpent-3-ényle, 2,6-diméthylhepta-1,5-diényle, 2-méthylpropényle.

7. Utilisation selon l'une quelconque des revendications précédentes, dans laquelle le composé est choisi dans le groupe constitué par les composés des formules 3, 14, 15, 16, 17, 18, 19, 20, 21, 22 et 23:

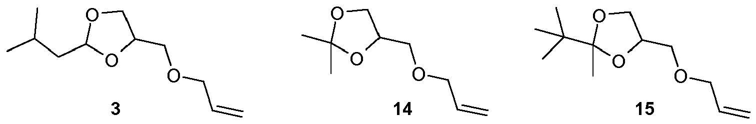

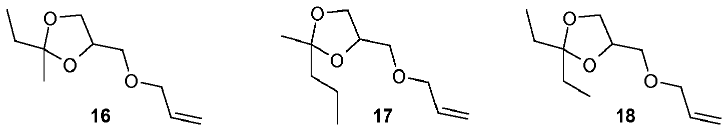

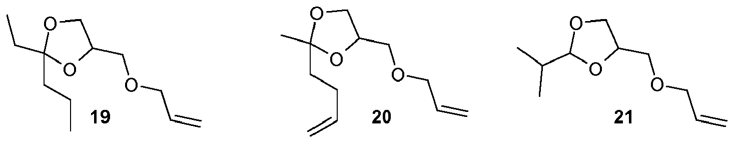

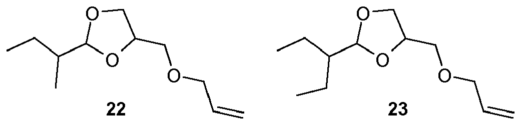

8. Utilisation selon l'une quelconque des revendications 1 à 3, dans laquelle R et R1 forment, conjointement avec l'atome de carbone auquel ils sont liés, un cycle qui comprend une ou deux liaisons doubles
et/ou
contient 1, 2 ou 3 atomes d'oxygène
et/ou,
au niveau des radicaux R et R1, est non substitué ou substitué par un ou plusieurs groupes - ramifiés ou non ramifiés - alkyle, alkényle, cycloalkyle, cycloalkényle, aryle, arylalkyle, alcoxyalkyle ou alcoxyaryle
et/ou
comprend, conjointement avec ses substituants existant le cas échéant, 5 à 30 atomes de carbone, de préférence 5 à 15 atomes de carbone.

9. Utilisation selon la revendication 8, dans laquelle le cycle est un carbocycle ou un hétérocycle.

10. Utilisation selon la revendication 9, dans laquelle le cycle est un carbocycle ou hétérocycle aliphatique.

11. Utilisation selon l'une quelconque des revendications 8 à 10, dans laquelle R et R1 forment, conjointement avec l'atome de carbone auquel ils sont liés, un cycle qui, au niveau des radicaux R et R1, est substitué par un ou plusieurs substituants choisis dans le groupe constitué par: les groupes méthyle, éthyle, n-propyle, isopropyle, n-butyle, sec-butyle, isobutyle, tert-butyle, n-pentyle, 2-pentyle, 3-pentyle, 2-méthylbutyle, 3-méthylbutyle, 3-méthylbut-2-yle, 2-méthylbut-2-yle, 2,2-diméthylpropyle, n-hexyle, 1-méthylpentyl, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle et 1-éthyl-2-méthylpropyle, but-3-ényle, 2,6-diméthylhept-5-ényle, 3,5-diméthylhex-4-ényle, 4-méthtylpent-3-ényle, 2,6-diméthylhepta-1,5-diényle, 2-méthylpropényle.

12. Utilisation selon l'une quelconque des revendications 8 à 11, dans laquelle le composé est choisi dans le groupe constitué par les composés ayant les formules 2, 4, 8, 1, 9, 10, 11, 7, 5, 6, 12 et 13:

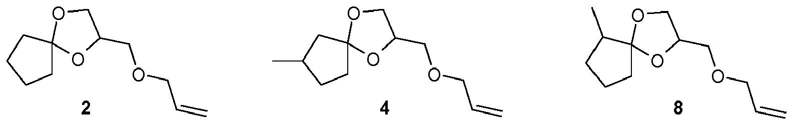

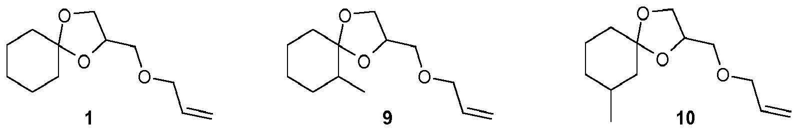

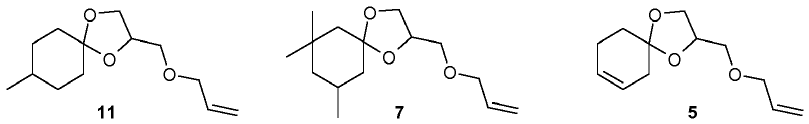

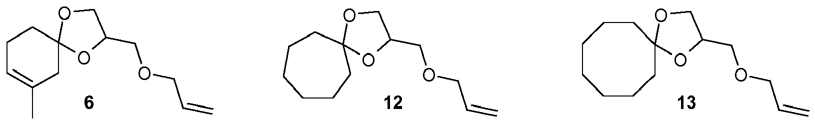

13. Composé de formule (I) 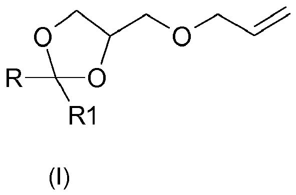 dans lequel,
- indépendamment l'un de l'autre, l'un des radicaux R et R1 signifie l'hydrogène et l'autre radical signifie prop-2-ène-1-yle, n-propyle ou isopropyle, ou un radical aliphatique non cyclique ayant 4 à 15 atomes de carbone qui est saturé ou insaturé et, en même temps, ramifié ou non ramifié,
ou,
- indépendamment l'un de l'autre, l'un des radicaux R et R1 signifie un groupe méthyle et l'autre radical signifie un radical aliphatique non cyclique insaturé ayant 2 à 15 atomes de carbone, qui est ramifié ou non ramifié,
ou,
- indépendamment l'un de l'autre, aussi bien R que R1 signifient un radical aliphatique non cyclique ayant 2 à 15 atomes de carbone, qui est respectivement saturé ou insaturé et en même temps respectivement ramifié ou non ramifié,
ou
- forment, conjointement avec l'atome de carbone auquel ils sont liés, un cycle ayant 5, 7 ou 8 atomes de carbone cycliques,
ou
- forment, conjointement avec l'atome de carbone auquel ils sont liés, un cycle ayant 6 atomes de carbone cycliques, le cycle étant substitué et/ou insaturé au niveau des radicaux R et R1,
ou
- forment, conjointement avec l'atome de carbone auquel ils sont liés, soit
(i) un cycle ayant exactement un atome d'oxygène cyclique, le cycle (a) possédant 6 atomes de carbone cycliques et l'atome d'oxygène n'est pas lié à l'atome de carbone auquel sont liés R et R1, ou (b) possédant 5, 7 ou 8 atomes de carbone cycliques, soit
(ii) un cycle ayant plusieurs atomes d'oxygène cycliques.

14. Composé selon la revendication 13, dans lequel R et R1 forment, conjointement avec l'atome de carbone auquel ils sont liés, un cycle qui
comprend 5 à 16 atomes de carbone dans l'ensemble
et/ou
comprend une ou deux liaisons doubles
et/ou
contient 1, 2 ou 3 atomes d'oxygène
et/ou,
au niveau des radicaux R et R1, est non substitué ou substitué par un ou plusieurs groupes - ramifiés ou non ramifiés - alkyle, alkényle, cycloalkyle, cycloalkényle, aryle, arylalkyle, alcoxyalkyle ou alcoxyaryle.

15. Composé selon la revendication 14, dans lequel le cycle est un carbocycle ou un hétérocycle.

16. Composé selon la revendication 15, dans lequel le cycle est un carbocycle ou hétérocycle aliphatique.

17. Composé selon l'une quelconque des revendications 13 à 16, dans lequel R et R1 forment, conjointement avec l'atome de carbone auquel ils sont liés, un cycle qui, au niveau des radicaux R et R1, est substitué par un ou plusieurs substituants choisis dans le groupe constitué par: les groupes méthyle, éthyle, n-propyle, isopropyle, n-butyle, sec-butyle, isobutyle, tert-butyle, n-pentyle, 2-pentyle, 3-pentyle, 2-méthylbutyle, 3-méthylbutyle, 3-méthylbut-2-yle, 2-méthylbut-2-yle, 2,2-diméthylpropyle, n-hexyle, 1-méthylpentyl, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle et 1-éthyl-2-méthylpropyle, but-3-ényle, 2,6-diméthylhept-5-ényle, 3,5-diméthylhex-4-ényle, 4-méthtylpent-3-ényle, 2,6-diméthylhepta-1,5-diényle, 2-méthylpropényle.

18. Composé selon l'une quelconque des revendications 13 à 17, le composé étant choisi dans le groupe constitué par les composés ayant les formules 2, 3, 4, 5, 6, 7, 8, 9, 10, 11, 12, 13, 18, 19, 20, 21, 22 et 23:

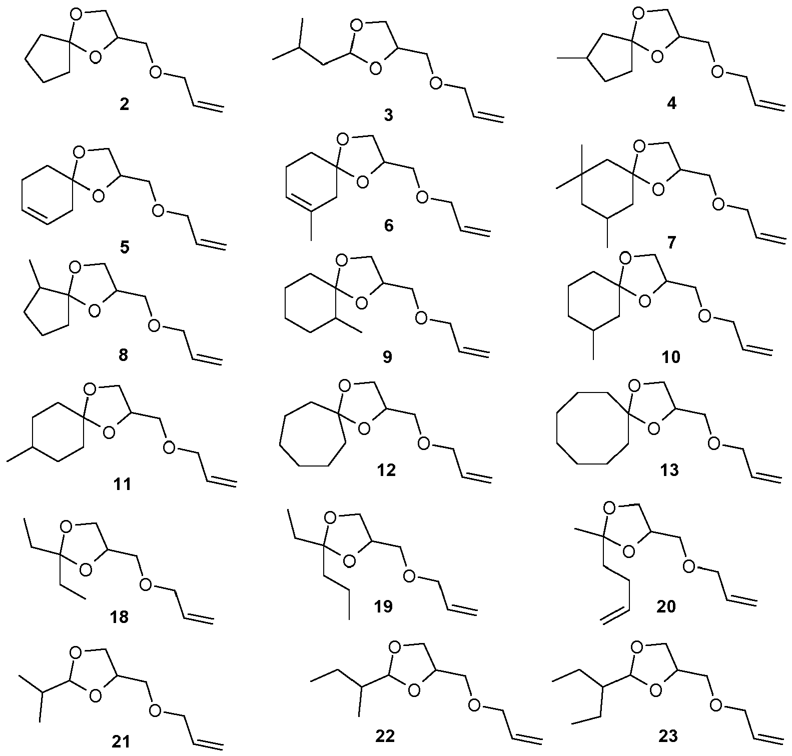

19. Composition de substances odoriférantes ou aromatisantes, comprenant:
a) un ou plusieurs composés tels que définis dans l'une quelconque des revendications précédentes 1 à 12 ou 13 à 18, ainsi que
b) une ou plusieurs autres substances odoriférantes ou aromatisantes,
(i) ladite ou lesdites autres substances odoriférantes ou aromatisantes conférant un(e) odeur et/ou goût ligneux/se, fruité(e) et/ou fleuri(e)
ou
(ii) ladite ou lesdites autres substances odoriférantes ou aromatisantes conférant un(e) autre odeur et/ou goût.

20. Composition de substances odoriférantes ou aromatisantes selon la revendication 19, dans laquelle le rapport pondéral de la quantité totale de composés tels que définis dans l'une quelconque des revendications précédentes 1 à 12 ou 13 à 18, à la quantité totale d'autres substances odoriférantes ou aromatisantes est compris entre 1 : 1000 et 1 : 0,5.

21. Composition de substances odoriférantes ou aromatisantes selon la revendication 19 ou 20, dans laquelle la quantité totale de composés tels que définis dans l'une quelconque des revendications précédentes 1 à 12 ou 13 à 18 est comprise entre 0,0001 et 99,9 % en poids, de préférence entre 0,001 et 99,5 % en poids, de manière particulièrement préférée entre 0,01 et 99 % en poids, respectivement par rapport à la quantité totale de la composition de substances odoriférantes ou aromatisantes.

22. Composition de substances odoriférantes ou aromatisantes selon l'une quelconque des revendications 19 à 21, dans laquelle la quantité totale utilisée du ou des composés tels que définis dans l'une quelconque des revendications précédentes 1 à 12 ou 13 à 18 est suffisante pour modifier l'odeur et/ou le goût des autres substances odoriférantes ou aromatisantes en direction de vert, herbacé, frais, fruité, fleuri, ligneux, doux, terreux, graisseux, métallique et balsamique, de préférence en direction de vert et/ou fruité, de manière préférée en direction de galbanum.

23. Utilisation d'une composition de substances odoriférantes ou aromatisantes selon l'une quelconque des revendications 19 à 22,
(A) en tant que composition de substances odoriférantes ou aromatisantes
(a)
(i) ayant une note de galbanum
et/ou
(ii) pour conférer, modifier et/ou intensifier une ou plusieurs notes olfactives et/ou gustatives choisies dans le groupe constitué par les notes verte, herbacée, fraîche, fruitée, fleurie, ligneuse, douce, terreuse, graisseuse, métallique et balsamique, de préférence au moins l'une des notes verte et/ou fruitée,
et/ou
(b)
(i) ayant une note fruitée et/ou fleurie,
et/ou
(ii) pour conférer, modifier et/ou intensifier une ou plusieurs notes olfactives et/ou gustatives choisies dans le groupe constitué par les notes ligneuse, fruitée et/ou fleurie,
et/ou
(c) ayant une note fleurie, fruitée, de type galbanum,
et/ou
(B) en tant que composant d'un article parfumé ou aromatisé.

24. Article parfumé ou aromatisé, comprenant:
- un ou plusieurs composés de formule (I), composés tels que définis dans l'une quelconque des revendications précédentes 13 à 18 ou une composition de substances odoriférantes ou aromatisantes selon l'une quelconque des revendications 19 à 22, de préférence en une quantité sensoriellement efficace,
et
- un ou plusieurs autres additifs, excipients et/ou principes actifs, de préférence deux, trois, quatre, cinq additifs, excipients et/ou principes actifs ou plus.

25. Article parfumé ou aromatisé selon la revendication 24, ledit article parfumé étant choisi dans le groupe constitué par:
les extraits de parfum, les eaux de parfum, les eaux de toilette, les après-rasages, les eaux de Cologne, les produits prérasage, les Splash Cologne, les lingettes rafraîchissantes parfumées, les produits de nettoyage acides, alcalins ou neutres, les produits à rafraîchir les textiles, les aides au repassage, les lessives liquides, les lessives en poudre, les produits de prétraitement de linge, les adoucissants de linge, les savons de lavage, les pastilles de lavage, les désinfectants, les désinfectants de surfaces, les produits de désodorisation de l'air, les aérosols, les cires et polis, les produits de toilette, les crèmes et lotions pour les mains, les crèmes et lotions pour les pieds, les crèmes et lotions dépilatoires, les crèmes et lotions après-rasage, les crèmes et lotions de bronzage, les produits de soin capillaire, les déodorants, les antiperspirants, les produits de la cosmétique décorative, les bougies, les huiles de lampe, les bâtonnets d'encens, les insecticides, les répulsifs et les carburants.

26. Article parfumé ou aromatisé selon la revendication 24 ou 25, dans lequel la quantité totale de composés tels que définis dans l'une quelconque des revendications précédentes 1 à 12 ou 13 à 18 est comprise entre 0,00001 et 10 % en poids, de préférence entre 0,0001 et 5 % en poids, de manière particulièrement préférée entre 0,001 et 2 % en poids, de la manière la plus préférée entre 0,005 et 1 % en poids, respectivement par rapport à la masse totale de l'article.

27. Procédé destiné à conférer, modifier et/ou intensifier une ou plusieurs notes olfactives et/ou gustatives choisies dans le groupe constitué par les notes verte, herbacée, fraîche, fruitée, fleurie, ligneuse, douce, terreuse, graisseuse, métallique et balsamique, de préférence au moins l'une des notes verte et/ou fruitée, de manière préférée une note de galbanum, comprenant les étapes suivantes:
- fournir un ou plusieurs composés tels que définis dans l'une quelconque des revendications précédentes 1 à 12 ou 13 à 18 ou une composition de substances odoriférantes ou aromatisantes selon l'une quelconque des revendications 19 à 22,
- fournir un article,
- mettre en contact ledit article avec une quantité sensoriellement efficace du ou des composés tels que définis dans l'une quelconque des revendications précédentes 1 à 12 ou 13 à 18 ou bien de la composition de substances odoriférantes ou aromatisantes selon l'une quelconque des revendications 19 à 22.

28. Procédé selon la revendication 27, dans lequel, après la mise en contact, (i) un article parfumé résulte ou (ii) est préparé à partir du produit qui est donc présent, l'article étant choisi dans le groupe constitué par:
les extraits de parfum, les eaux de parfum, les eaux de toilette, les après-rasages, les eaux de Cologne, les produits prérasage, les Splash Cologne, les lingettes rafraîchissantes parfumées, les produits de nettoyage acides, alcalins ou neutres, les produits à rafraîchir les textiles, les aides au repassage, les lessives liquides, les lessives en poudre, les produits de prétraitement de linge, les adoucissants de linge, les savons de lavage, les pastilles de lavage, les désinfectants, les désinfectants de surfaces, les produits de désodorisation de l'air, les aérosols, les cires et polis, les produits de toilette, les crèmes et lotions pour les mains, les crèmes et lotions pour les pieds, les crèmes et lotions dépilatoires, les crèmes et lotions après-rasage, les crèmes et lotions de bronzage, les produits de soin capillaire, les déodorants, les antiperspirants, les produits de la cosmétique décorative, les bougies, les huiles de lampe, les bâtonnets d'encens, les insecticides, les répulsifs et les carburants.
